(19) Europäisches Patentamt
European Patent Office
Office européen des brevets

(11) **EP 3 751 278 B1**

(12) **FASCICULE DE BREVET EUROPEEN**

(45) Date de publication et mention de la délivrance du brevet:
**16.06.2021 Bulletin 2021/24**

(51) Int Cl.:
**G01N 29/24** *(2006.01)*      **A61B 5/00** *(2006.01)*
**G01N 21/17** *(2006.01)*      **G01N 21/45** *(2006.01)*

(21) Numéro de dépôt: **20179391.6**

(22) Date de dépôt: **10.06.2020**

(54) **SYSTÈME D'IMAGERIE ACOUSTO-OPTIQUE**

**AKUSTISCH-OPTISCHES BILDGEBUNGSSYSTEM**

**SYSTEM FOR ACOUSTO-OPTICAL IMAGING**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.06.2019 FR 1906281**

(43) Date de publication de la demande:
**16.12.2020 Bulletin 2020/51**

(73) Titulaire: **Commissariat à l'Energie Atomique et aux Energies Alternatives**
**75015 Paris (FR)**

(72) Inventeurs:
• **VERDANT, Arnaud**
**38054 GRENOBLE CEDEX 9 (FR)**
• **GUICQUERO, William**
**38054 GRENOBLE CEDEX 9 (FR)**

(74) Mandataire: **Cabinet Beaumont**
**4, Place Robert Schuman**
**B.P. 1529**
**38025 Grenoble Cedex 1 (FR)**

(56) Documents cités:
**US-A1- 2016 327 904**

• **EMILIE BENOIT A LA GUILLAUME ET AL: "Acousto-optical coherence tomography with a digital holographic detection scheme", OPTICS LETTERS, OPTICAL SOCIETY OF AMERICA, US, vol. 37, no. 15, 1 août 2012 (2012-08-01), pages 3216-3218, XP001577522, ISSN: 0146-9592, DOI: 10.1364/OL.37.003216 [extrait le 2012-07-26]**

**Description**

Domaine technique

La présente demande concerne le domaine de l'imagerie acousto-optique, notamment pour des applications médicales.

Technique antérieure

Dans un milieu biologique tel qu'un tissu humain ou animal, la lumière visible se propage selon un régime de diffusion. Dans ce régime, les photons empruntent des chemins optiques multiples, de sorte que leurs agencements relatifs sont modifiés tout au long de la propagation de la lumière à travers le milieu. Il est alors impossible, avec un capteur d'images standard, d'acquérir une image fidèle du milieu traversé par la lumière.

Pour remonter à des informations d'absorption localisée dans le milieu, on a déjà proposé des techniques dites d'imagerie acousto-optique, dans lesquelles le milieu observé est éclairé par un faisceau de lumière cohérente, typiquement un faisceau laser, et une partie du milieu observé est soumise à une onde acoustique, typiquement une onde acoustique ultrasonore. La vibration des diffuseurs le long du chemin de propagation de l'onde acoustique, aussi appelé zone de marquage acoustique ou encore colonne acoustique de marquage, module alors la phase des photons diffusés. En sortie du milieu, on retrouve des photons porteurs de la modulation acoustique, appelés photons marqués, correspondant aux photons passés par la zone de marquage acoustique, et des photons non porteurs de la modulation acoustique, appelés photons non marqués, correspondant aux photons qui ne sont pas passés par la zone de marquage acoustique. On fait alors interférer le faisceau lumineux issu du milieu, appelé faisceau objet, avec un faisceau de référence issu de la même source lumineuse mais n'ayant pas traversé le milieu diffusant. Ceci permet de démoduler le faisceau objet et de ramener la pulsation des photons marqués à la fréquence de l'onde acoustique. Un capteur d'images est alors utilisé pour mesurer le signal lumineux modulé à la fréquence acoustique et en déduire des informations quant à l'absorption de la lumière dans la zone de marquage.

Un système d'imagerie acousto-optique de l'art antérieur est par exemple décrit dans la demande de brevet US-A-2016327904, qui divulgue un système d'imagerie, comportant : une source de lumière cohérente fournissant un faisceau objet et un faisceau de référence; un dispositif de modulation adapté à moduler tout ou partie du faisceau objet par un signal de modulation; un capteur d'images agencé pour recevoir une figure d'interférence résultant d'une combinaison du faisceau objet et du faisceau de référence; et un dispositif de démodulation de la partie modulée du faisceau objet, le système étant configuré pour, lors d'une phase de mesure appliquer au signal de modulation, par l'intermédiaire du dispositif de modulation, une première séquence pseudo-aléatoire de sauts de phase et appliquer simultanément à la partie modulée du faisceau objet, par l'intermédiaire du dispositif de démodulation, une deuxième séquence pseudo-aléatoire de sauts de phase, dans lequel les première et deuxième séquences de sauts de phase sont corrélées.

Il serait souhaitable de pouvoir disposer d'un système d'imagerie acousto-optique, ce système palliant tout ou partie des inconvénients des systèmes d'imagerie acousto-optique connus.

Résumé de l'invention

Pour cela, un mode de réalisation prévoit un système d'imagerie, comportant :

- une source de lumière cohérente fournissant un faisceau objet et un faisceau de référence ;
- un dispositif de modulation adapté à moduler tout ou partie du faisceau objet par un signal de modulation ;
- un capteur d'images agencé pour recevoir une figure d'interférence résultant d'une combinaison du faisceau objet et du faisceau de référence ; et
- un dispositif de démodulation de la partie modulée du faisceau objet,

le système étant configuré pour, lors d'une phase de mesure :

- appliquer au signal de modulation, par l'intermédiaire du dispositif de modulation, une première séquence pseudo-aléatoire de sauts d'un paramètre choisi parmi la phase, la fréquence et l'amplitude ; et
- appliquer simultanément à la partie modulée du faisceau objet, par l'intermédiaire du dispositif de démodulation, une deuxième séquence pseudo-aléatoire de sauts dudit paramètre,

dans lequel les première et deuxième séquences de sauts dudit paramètre sont non corrélées.

Selon un mode de réalisation, le système est configuré pour, lors d'une phase d'acquisition, mettre en oeuvre M phases de mesure successives, M étant un entier supérieur ou égal à 2, et, à chaque phase de mesure, acquérir, via le capteur d'images, une valeur représentative du champ complexe de la partie modulée du faisceau objet.

Selon un mode de réalisation, le système comporte un dispositif de traitement configuré pour mettre en oeuvre une étape de reconstruction, à partir des M valeurs acquises lors de la phase d'acquisition, d'un jeu de L valeurs représentatives de l'absorption lumineuse à L positions distinctes sur le trajet optique de la partie modulée du faisceau objet, L étant un entier supérieur ou égal à 2.

Selon un mode de réalisation, L est supérieur à M.

Selon un mode de réalisation, le dispositif de modulation est un dispositif de modulation acousto-optique.

Selon un mode de réalisation, le dispositif de modulation comprend un transducteur ultrasonore, le signal de modulation étant une onde ultrasonore appliquée par le transducteur ultrasonore à une partie d'un échantillon à analyser, placé sur le trajet optique du faisceau objet.

Selon un mode de réalisation, le dispositif de modulation comprend une pluralité de transducteurs ultrasonores agencés en barrette ou en matrice, et, lors de chaque phase de mesure, l'onde ultrasonore de modulation est émise par fraction par les différents transducteurs selon une fonction pseudo-aléatoire $\gamma$.

Selon un mode de réalisation, le dispositif de modulation comprend un modulateur acousto-optique placé sur le trajet optique du faisceau objet en amont d'une scène à analyser.

Selon un mode de réalisation, le dispositif de démodulation est un dispositif de démodulation électronique intégré au capteur d'images.

Selon un mode de réalisation, le dispositif de démodulation comprend un modulateur acousto-optique placé sur le trajet optique du faisceau de référence, en amont du capteur d'images.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente de façon schématique un exemple d'un système d'imagerie acousto-optique ;

la figure 2 est un diagramme illustrant le fonctionnement du système de la figure 1 ;

la figure 3 représente de façon schématique un autre exemple d'un système d'imagerie acousto-optique ;

la figure 4 est un diagramme illustrant le fonctionnement du système de la figure 3 ;

la figure 5 représente de façon schématique un exemple d'un système d'imagerie acousto-optique selon un premier mode de réalisation ;

la figure 6 représente de façon schématique un exemple d'un système d'imagerie acousto-optique selon un deuxième mode de réalisation ;

la figure 7 représente de façon schématique un exemple d'un système d'imagerie acousto-optique selon un troisième mode de réalisation ;

la figure 8 illustre le fonctionnement d'une variante de réalisation d'un système d'imagerie acousto-optique ; et

la figure 9 illustre plus en détail le fonctionnement de la variante de la figure 8.

Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les applications d'imagerie pouvant être mises en oeuvre à partir des capteurs décrits n'ont pas été détaillées, les modes de réalisation décrits étant compatibles avec les applications usuelles d'imagerie acousto-optique, dans le domaine médical ou dans d'autres domaines.

Sauf précision contraire, lorsque l'on fait référence à deux éléments connectés entre eux, cela signifie directement connectés sans éléments intermédiaires autres que des conducteurs, et lorsque l'on fait référence à deux éléments reliés ou couplés entre eux, cela signifie que ces deux éléments peuvent être connectés ou être reliés ou couplés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures, étant entendu que, en pratique, les dispositifs décrits peuvent être orientés différemment.

Sauf précision contraire, les expressions "environ", "approximativement", "sensiblement", et "de l'ordre de" signifient à 10 % près, de préférence à 5 % près.

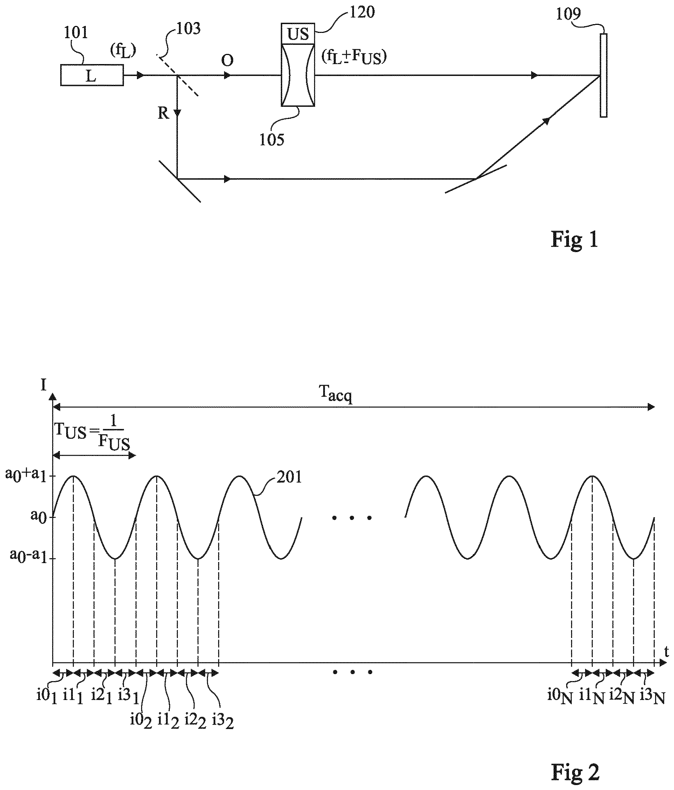

La figure 1 représente de façon schématique un exemple d'un système d'imagerie acousto-optique.

Le système de la figure 1 comprend une source lumineuse 101 (L) adaptée à générer un faisceau lumineux cohérent de fréquence $f_L$, par exemple une source laser. Le système comprend en outre un séparateur 103, par exemple une lame 103 partiellement transparente et partiellement réfléchissante placée à 45 degrés par rapport à la direction d'émission de la source 101, permettant de diviser le faisceau produit par la source 101 en un faisceau objet O et un faisceau de référence R. Le système est agencé de façon que le faisceau objet O illumine un objet ou un échantillon 105 à analyser, et que le faisceau de référence R ne passe pas par l'objet 105. Le faisceau de référence et le faisceau objet réfléchi ou transmis par l'objet 105 sont ensuite projetés sur un capteur d'images 109 comportant une matrice de pixels (non détaillée), de manière à produire une figure d'interférence dans le plan d'acquisition du capteur.

Dans le système de la figure 1, une partie de l'objet ou échantillon 105 est excitée par une onde acoustique sinusoïdale de fréquence $F_{US}$, par exemple comprise entre 1 et 15 MHz, générée par exemple par un transducteur ultrasonore 120. Il en résulte que la fréquence des rayons du faisceau objet passant par la partie excitée de l'échantillon est décalée à la valeur $f_L+F_{US}$ ou $f_L-F_{US}$, alors que la fréquence des rayons du faisceau objet passant par les parties non-excitées de l'échantillon reste à la valeur $f_L$. Ce décalage fréquentiel permet de "marquer" les photons du faisceau objet passés par la partie excitée de l'échantillon, appelée zone de marquage, par rapport aux autres photons du faisceau objet. On cherche alors à mesurer l'énergie portée par les photons marqués, pour en déduire des informations quant à l'absorption lumineuse dans la zone de marquage.

La figure 2 est un diagramme illustrant plus en détail le fonctionnement du système de la figure 1.

Le diagramme de la figure 2 comprend une courbe 201 représentant l'évolution en fonction du temps t (en abscisse) de l'intensité lumineuse I (en ordonnée) reçue par un pixel du capteur 109 du système de la figure 1, lorsque le capteur 109 est éclairé par le faisceau objet et le faisceau de référence combinés.

En fonctionnement, le capteur 109 voit une figure d'interférence aussi appelée figure de speckle, constituée de taches résultant des interférences constructives ou destructives du faisceau objet diffus. Chaque tache possède un module relativement constant sur toute l'étendue de la tache, dépendant en premier lieu de l'absorption des photons dans la zone de marquage, et une phase aléatoire dépendant des chemins optiques empruntés par la lumière dans le milieu. L'échantillonnage spatial des taches est de préférence tel que chaque tache couvre au moins un pixel. L'interférence avec le faisceau de référence conduit à la création de franges d'interférences. On peut contrôler l'angle d'incidence du faisceau de référence sur le capteur afin d'obtenir la période spatiale interfranges souhaitée. La figure d'interférence vue par le capteur 109 présente une fréquence de battement égale à la fréquence de modulation acoustique $F_{US}$. Ainsi, comme cela apparaît sur la figure 2, l'intensité I reçue par chaque pixel évolue de façon sinusoïdale autour d'une valeur moyenne ou composante continue $a_0$, avec une amplitude de crête de valeur $a_1$, à une fréquence égale à la fréquence de modulation acoustique $F_{US}$ du système. Les intensités lumineuses reçues par différents pixels du capteur 109 peuvent avoir des composantes continues $a_0$ distinctes et/ou des amplitudes $a_1$ distinctes, mais fluctuent toutes de façon sinusoïdale à la fréquence de battement $F_{US}$, avec des déphasages propres.

Chaque pixel du capteur 109 comprend une photodiode (non représentée) fournissant un courant représentatif de l'intensité lumineuse reçue par le pixel.

Dans cet exemple, la mesure de la partie marquée du faisceau objet s'effectue pendant une période d'acquisition $T_{acq}$ de durée $N*T_{US}$, où $T_{US}$ désigne la période de modulation acoustique du système, égale à $1/F_{US}$, et où N est un entier, supérieur ou égal à 1, par exemple compris entre 100 et 10000.

Chaque pixel comprend un circuit d'intégration configuré pour, à chaque période de modulation acoustique $T_{US}$ de la période d'acquisition $T_{acq}$, intégrer le photocourant délivré par la photodiode successivement dans K=4 capacités C0, C1, C2 et C3 distinctes (non représentées). Ce cycle d'intégration et d'échantillonnage à quatre phases est répété successivement N fois pendant la période $T_{acq}$, étant entendu que les capacités d'intégration C0, C1, C2 et C3 du pixel ne sont pas réinitialisées entre le début et la fin de la période $T_{acq}$. L'accumulation des échantillons sur un nombre N élevé de périodes du signal de modulation acoustique permet d'améliorer le rapport signal sur bruit de la mesure. Le nombre N est de préférence choisi de façon que la durée de la phase d'acquisition $T_{acq}$ reste inférieure à une durée caractéristique de décorrélation du milieu, par exemple comprise entre 0,1 et 5 ms, par exemple de l'ordre de 1 ms, correspondant au temps au-delà duquel l'amplitude et la phase du signal mesuré risquent de varier du fait de variations physiologiques du milieu observé (par exemple du fait d'un mouvement de plaquettes sanguines).

Sur la figure 2, pour chacune des N périodes $T_{US}$ de la phase d'acquisition $T_{acq}$, on a désigné par les références

i0$_p$, i1$_b$, i2$_p$ et i3$_p$ les quatre périodes d'intégration successives du photocourant fourni par la photodiode du pixel, respectivement dans les quatre capacités C0, C1, C2 et C3 du pixel, où p est un entier allant de 1 à N désignant le rang de la période de modulation acoustique T$_{US}$ considérée dans la phase d'acquisition T$_{acq}$. Dans cet exemple, les périodes d'intégration i0$_p$, i1$_b$, i2$_p$ et i3$_p$ ont toutes sensiblement la même durée, de l'ordre de T$_{US}$/4.

A la fin de la phase d'acquisition T$_{acq}$, chaque pixel du capteur fournit quatre valeurs I0, I1, I2 et I3 représentatives respectivement des tensions aux bornes des capacités C0, C1, C2 et C3 du pixel.

A partir de ces quatre valeurs, le champ complexe E$_{OM}$ de la partie du faisceau objet marquée par la modulation acoustique peut être déterminé, pour chaque pixel du capteur, par la formule E$_{OM}$ = (I0-I2) + i(I1-I3), i étant l'unité imaginaire. La composante I0-I2 correspond à la partie réelle du champ E$_{OM}$, et la composante I1-I3 correspond à la partie imaginaire du champ E$_{OM}$. La phase P$_{OM}$ et l'amplitude A$_{OM}$ du faisceau objet marqué reçu par chaque pixel du capteur peuvent être déterminées par les formules suivantes :

[Math 1]

$$P_{OM} = \arctan(\frac{I1 - I3}{I0 - I2})$$

et

[Math 2]

$$A_{OM} = \sqrt{(I0 - I2)^2 + (I1 - I3)^2}$$

La connaissance de l'amplitude A$_{OM}$ du faisceau objet marqué permet en particulier d'obtenir des informations quant à l'absorption lumineuse dans la zone de marquage acoustique, par exemple en vue de détecter la présence éventuelle d'un corps absorbant, par exemple une tumeur, dans la zone de marquage.

On notera que la technique d'acquisition décrite en relation avec les figures 1 et 2 permet uniquement de déterminer l'absorption moyenne au sein de la zone de marquage, mais ne permet pas d'obtenir d'informations sur l'absorption dans des régions localisées de la zone de marquage.

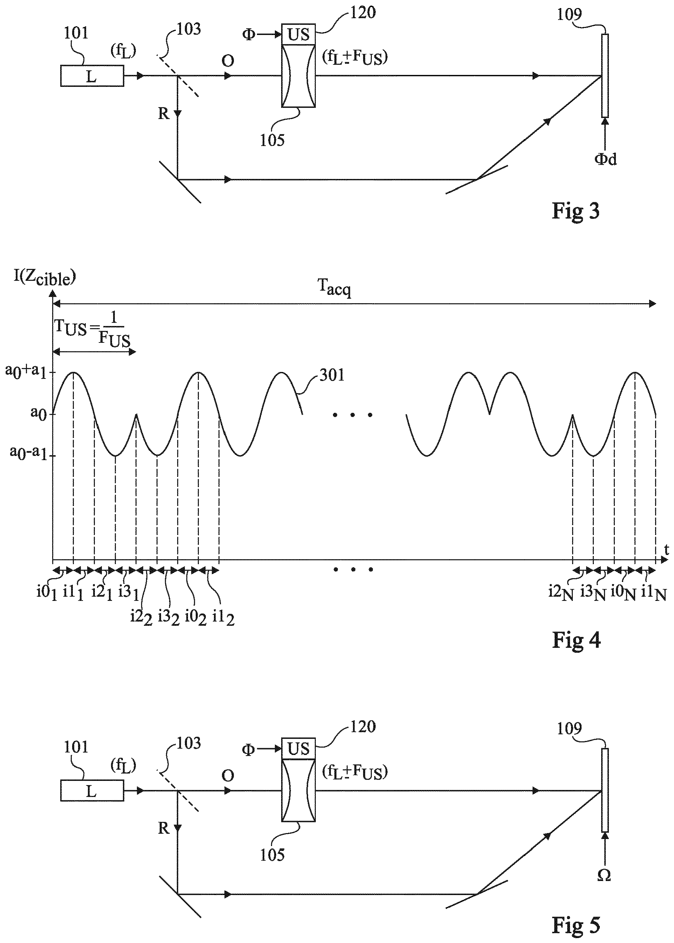

La figure 3 représente de façon schématique un autre exemple d'un système d'imagerie acousto-optique, permettant de déterminer l'absorption dans des régions localisées de la zone de marquage.

Le système de la figure 3 diffère du système de la figure 1 principalement en ce que, dans le système de la figure 3, on applique à l'onde acoustique de marquage une séquence Φ de sauts de phase présentant un motif aléatoire ou pseudo-aléatoire, appelée séquence de modulation. A titre d'exemple, à chaque nouvelle période de modulation acoustique T$_{US}$, un saut de phase de valeur 0 ou π, choisie de façon aléatoire ou pseudo-aléatoire, est appliqué à l'onde acoustique de marquage. Au niveau du capteur 109, après interférence du faisceau objet avec le faisceau de référence, le signal est échantillonné selon la méthode de démodulation quatre phases exposée en relation avec la figure 2, adaptée pour tenir compte de la séquence Φ de sauts de phase appliquée à l'onde acoustique de marquage.

La figure 4 est un diagramme illustrant plus en détail le fonctionnement du système de la figure 3.

Le diagramme de la figure 4 comprend une courbe 301 représentant l'évolution, en fonction du temps t, en abscisse, de la contribution I(Z$_{cible}$), en ordonnée, d'une position localisée Z$_{cible}$ de la zone de marquage, au signal lumineux objet reçu par le capteur 109 après interférence avec le signal de référence. Dans cet exemple, la position Z$_{cible}$ correspond à une position dans l'axe de propagation de l'onde acoustique de modulation.

L'intensité I(Z$_{cible}$) évolue de façon sinusoïdale autour d'une valeur moyenne ou composante continue a$_0$, avec une amplitude de crête de valeur a$_1$, à une fréquence égale à la fréquence de modulation acoustique F$_{US}$ du système. Dans cet exemple, à chaque nouvelle période T$_{US}$ de la phase d'acquisition T$_{acq}$, l'intensité I(Z$_{cible}$) subit un saut de phase de 0 ou π. La séquence Φd de sauts de phase subie par l'intensité I(Z$_{cible}$) correspond à la séquence de sauts de phase Φ appliquée à l'onde acoustique de marquage, retardée d'un délai Δt=Z$_{cible}$/υ$_{US}$, υ$_{US}$ désignant la vitesse de propagation de l'onde acoustique de marquage dans le milieu observé.

Dans chaque pixel du capteur, le circuit d'intégration est configuré pour, à chaque période de modulation acoustique T$_{US}$ de la période d'acquisition T$_{acq}$, intégrer le photocourant délivré par la photodiode successivement dans les quatre capacités C0, C1, C2 et C3, en tenant compte de la séquence de sauts de phase retardée Φd = Φ(t-Δt), appelée séquence de démodulation, pour déterminer l'ordre dans lequel les capacités C0, C1, C2 et C3 sont connectées à la photodiode. Plus particulièrement, dans cet exemple, à chaque saut de phase de valeur π dans la séquence Φd, l'ordre d'intégration du photocourant dans les capacités C0 et C2 d'une part et C1 et C3 d'autre part est inversé.

A la fin de la phase d'acquisition T$_{acq}$, chaque pixel du capteur fournit quatre valeurs I0, I1, I2 et I3 représentatives respectivement des tensions aux bornes des capacités C0, C1, C2 et C3 du pixel. Les valeurs I0, I1, I2 et I3 sont représentatives principalement de la contribution au signal lumineux objet reçu par le pixel, de la position de la zone de marquage pour laquelle le motif de sauts de phase $\Phi$d est corrélé au signal de modulation acoustique, c'est-à-dire la position $Z_{cible} = \upsilon_{US}*\Delta t$.

A partir de ces quatre valeurs, le champ complexe $E_{OM}$ de la partie du faisceau objet marquée par la modulation acoustique à la position $Z_{cible}$ de la zone de marquage peut être déterminé par la formule $E_{OM} = (I0-I2) + j (I1-I3)$. La composante I0-I2 correspond à la partie réelle du champ $E_{OM}$, et la composante I1-I3 correspond à la partie imaginaire du champ $E_{OM}$. La phase $P_{OM}$ et l'amplitude $A_{OM}$ du faisceau objet marqué reçu en provenance de la position $Z_{cible}$ peuvent être déterminées par les mêmes formules Math 1 et Math 2 que dans l'exemple des figures 1 et 2.

La mesure peut être réitérée en modifiant le retard $\Delta t$ entre la séquence $\Phi$ de sauts de phase appliquée au signal de modulation acoustique et la séquence $\Phi$d appliquée au capteur pendant la phase d'acquisition $T_{acq}$, de façon à mesurer le champ $E_{OM}$ en provenance d'une autre position cible $Z_{cible}$ dans la zone de marquage. Plus particulièrement, si l'on désigne par L la résolution axiale souhaitée, c'est-à-dire le nombre souhaité de points de mesure ayant des positions $Z_{cible}$ différentes dans la colonne de marquage acoustique (i.e. le long de l'axe principal de propagation du faisceau acoustique de marquage), la mesure doit être réitérée L fois.

La figure 5 représente de façon schématique un exemple d'un système d'imagerie acousto-optique selon un premier mode de réalisation.

Le système de la figure 5 comprend les mêmes éléments que le système de la figure 3, agencés sensiblement de la même manière, et diffère du système de la figure 3 principalement par son mode de fonctionnement.

Dans le mode de réalisation de la figure 5, on prévoit de reconstruire un profil d'absorption de la colonne de marquage, de résolution axiale L, à partir d'une série de M mesures, avec L et M entiers supérieurs ou égaux à 2. Lors de chaque mesure, une première séquence de sauts de phase $\Phi$ aléatoire ou pseudo-aléatoire, appelée séquence de modulation, est appliquée au signal acoustique de marquage, de façon identique ou similaire à ce qui a été décrit dans l'exemple des figures 3 et 4. Lors de la phase d'acquisition $T_{acq}$, chaque pixel du capteur 109 applique au signal mesuré une deuxième séquence de sauts de phase $\Omega$ aléatoire ou pseudo-aléatoire, appelée séquence de démodulation. A titre d'exemple, à chaque nouvelle période de modulation acoustique $T_{US}$ de la phase d'acquisition $T_{acq}$, un saut de phase de valeur 0 ou $\pi$ est appliqué au signal mesuré. L'ensemble des N valeurs de sauts de phase appliquées pendant la phase d'acquisition $T_{acq}$ définit la séquence $\Omega$. Dans chaque pixel du capteur, le circuit d'intégration est configuré pour, à chaque période de modulation acoustique $T_{US}$ de la phase d'acquisition $T_{acq}$, intégrer le photocourant délivré par la photodiode successivement dans les quatre capacités C0, C1, C2 et C3, en tenant compte de la séquence $\Omega$ pour déterminer l'ordre dans lequel les capacités C0, C1, C2 et C3 sont connectées à la photodiode. Plus particulièrement, dans cet exemple, à chaque saut de phase de valeur $\pi$ dans la séquence $\Omega$, l'ordre d'intégration du photocourant dans les capacités C0 et C2 d'une part et C1 et C3 d'autre part est inversé.

A la différence de l'exemple des figures 3 et 4 dans lequel la séquence $\Phi$ de modulation de la phase du faisceau acoustique de marquage et la séquence de démodulation $\Phi$d contrôlant les phases d'échantillonnage appliquée par le capteur 109 lors de la phase d'acquisition $T_{acq}$ présentent un pic de corrélation défini pour une position spécifique dans la colonne de marquage, dans l'exemple de la figure 5, les séquences de modulation $\Phi$ et de démodulation $\Omega$ sont non corrélées. Les séquences $\Phi$ et $\Omega$ correspondent par exemple à deux tirages aléatoires ou pseudo-aléatoires indépendants de séquences binaires, par exemple des tirages aléatoires suivant une distribution de Bernoulli équiprobable, dans lesquels un état de la séquence binaire correspond à une période de modulation $T_{US}$. Deux nouveaux tirages aléatoires ou pseudo-aléatoires indépendants des tirages précédents, c'est-à-dire non corrélés avec les tirages précédents, peuvent être appliqués à chacune des M mesures de la phase d'acquisition du profil d'absorption de l'échantillon (soit 2*M tirages distincts et non corrélés entre eux pour l'acquisition d'un profil d'absorption de L valeurs). Chaque mesure résulte alors d'une contribution variable des différents points de la colonne de marquage de l'échantillon.

Les inventeurs ont montré qu'un modèle d'acquisition peut être construit, permettant de modéliser les mesures comme une projection du profil d'absorption recherché au travers d'une matrice de passage.

Le signal lumineux s(t) vu au niveau d'un pixel du capteur 109, résultant de l'interférence entre le faisceau objet (signal $E_s(t)$) et le faisceau de référence (signal $E_r(t)$) est donné par l'équation suivante :

[Math 3]

$$s(t) = |E_r(t) + E_s(t)|^2$$

Avec :

[Math 4]

$$E_r(t) = A_R e^{i\omega_L t}$$

Et :

[Math 5]

$$E_s(t) = A_D e^{i\omega_L t} + \int_z a_M(z) e^{i(\omega_L t + \omega_{US}(t - z/v_{US}) + \pi\Phi(t - z/v_{US} + \delta))} dz$$

$$+ \int_z a_M(z) e^{i(\omega_L t - \omega_{US}(t - z/v_{US}) - \pi\Phi(t - z/v_{US} + \delta))} dz$$

Où $A_R$, $A_D$ et $a_M$ sont les amplitudes complexes des champs optiques respectivement du bras de référence, des photons non marqués du bras objet, et des photons marqués du bras objet, t est une variable temps, z désigne la position le long de la colonne de marquage acoustique de l'échantillon ($a_M(z)$ correspond à l'absorption de l'échantillon à la position z de la colonne de marquage), $v_{US}$ désigne la vitesse de propagation de l'onde acoustique dans l'échantillon, $\omega_L$ et $\omega_{US}$ désignent respectivement la pulsation de l'onde lumineuse émise par la source 101 et la pulsation de l'onde acoustique de marquage, $\Phi$ désigne la séquence binaire de modulation de la phase de l'onde acoustique de marquage, et $\delta$ est un terme traduisant le fait que la phase du signal modulant peut ne pas être nulle à l'origine.

Le signal s(t) peut alors être exprimé comme suit :

[Math 6]

$$s(t) = \left( \left| e^{i\omega_L t} \right| . \left| A_D + A_R \right. \right.$$

$$+ \int_z a_M(z) e^{i(\omega_{US}(t - z/v_{US}) + \pi\Phi(t - z/v_{US} + \delta))} dz$$

$$\left. + \int_z a_M(z) e^{i(-\omega_{US}(t - z/v_{US}) - \pi\Phi(t - z/v_{US} + \delta))} dz \right| \Big)^2$$

Puis :

[Math 7]

$$s(t) = \left| A_D + A_R + 2 \int_z a_M(z) \cos\left(\omega_{US}(t - z/v_{US}) + \pi\Phi(t - z/v_{US} + \delta)\right) dz \right|^2$$

Pour chacune des M mesures de la phase d'acquisition du profil d'absorption, on mesure au niveau du capteur, par la technique de démodulation quatre phases exposée ci-dessus, un terme I=I0-I2 et un terme Q=I1-I3, avec :

[Math 8]

$$I = \sum_{p=1}^{N} \Pi(p) \left( \int_{pT_{US}}^{pT_{US}+T_{US}/4} s(t)dt - \int_{pT_{US}+T_{US}/2}^{pT_{US}+3T_{US}/4} s(t)dt \right)$$

Et :

[Math 9]

$$Q = \sum_{p=1}^{N} \Pi(p) \left( \int_{pT_{US}+T_{US}/4}^{pT_{US}+T_{US}/2} s(t)dt - \int_{pT_{US}+3T_{US}/4}^{pT_{US}+T_{US}} s(t)dt \right)$$

Avec :

[Math 10]

$$\Pi(p) = 2 \left( \Omega(p) - \frac{1}{2} \right)$$

Où $\Omega$ désigne la séquence binaire de démodulation appliquée par le capteur 109.
Le terme Q peut s'exprimer comme suit :

[Math 11]

$$Q = \sum_{p=1}^{N} \Pi(p) \left( \int_{pT_{US}+T_{US}/4}^{pT_{US}+T_{US}/2} \left( Re\left( A_D + A_R \right. \right. \right.$$

$$\left. \left. + 2\int_z a_M(z)cos(\omega_{US}(t - z/v_{US}) + \pi\Phi(t - z/v_{US} + \delta))dz \right) \right)^2 dt$$

$$- \int_{pT_{US}+3T_{US}/4}^{pT_{US}+T_{US}} \left( Re\left( A_D + A_R \right. \right.$$

$$\left. \left. + 2\int_z a_M(z)cos(\omega_{US}(t - z/v_{US}) + \pi\Phi(t - z/v_{US} + \delta))dz \right) \right)^2 dt$$

$$+ \int_{pT_{US}+T_{US}/4}^{pT_{US}+T_{US}/2} \left( Im\left( A_D + A_R \right. \right.$$

$$\left. \left. + 2\int_z a_M(z)cos(\omega_{US}(t - z/v_{US}) + \pi\Phi(t - z/v_{US} + \delta))dz \right) \right)^2 dt$$

$$- \int_{pT_{US}+3T_{US}/4}^{pT_{US}+T_{US}} \left( Im\left( A_D + A_R \right. \right.$$

$$\left. \left. + 2\int_z a_M(z)cos(\omega_{US}(t - z/v_{US}) + \pi\Phi(t - z/v_{US} + \delta))dz \right) \right)^2 dt \right)$$

Où Re et Im désignent respectivement la partie réelle et la partie imaginaire d'un nombre complexe.

Les termes ne comprenant pas la composante $A_R$ du bras de référence peuvent être négligés, l'énergie apportée par ce bras étant largement supérieure à l'énergie apportée par les parties marquées et non marquées du bras objet.

L'équation Math 11 peut alors être simplifiée comme suit :

[Math 12]

$$Q = \sum_{p=1}^{N} \Pi(p) \left( \int_{pT_{US}+T_{US}/4}^{pT_{US}+T_{US}/2} 4Re(A_R) \left( \int_z Re\big(a_M(z)G(t,z)\big)dz \right) dt \right.$$

$$- \int_{pT_{US}+3T_{US}/4}^{pT_{US}+T_{US}} 4Re(A_R) \left( \int_z Re\big(a_M(z)G(t,z)\big)dz \right) dt$$

$$+ \int_{pT_{US}+T_{US}/4}^{pT_{US}+T_{US}/2} 4Im(A_R) \left( \int_z Im\big(a_M(z)G(t,z)\big)dz \right) dt$$

$$\left. - \int_{pT_{US}+3T_{US}/4}^{pT_{US}+T_{US}} 4Im(A_R) \left( \int_z Im\big(a_M(z)G(t,z)\big)dz \right) dt \right)$$

Avec :

[Math 13]

$$G(t,z) = cos\big(\omega_{US}(t - z/v_{US}) + \pi\Phi(t - z/v_{US} + \delta)\big)$$

De façon similaire, le terme I peut être exprimé comme suit :

[Math 14]

$$I = \sum_{p=1}^{N} \Pi(p) \left( \int_{pT_{US}}^{pT_{US}+T_{US}/4} 4Re(A_R) \left( \int_z Re\big(a_M(z)G(t,z)\big)dz \right) dt \right.$$

$$- \int_{pT_{US}+T_{US}/2}^{pT_{US}+3T_{US}/4} 4Re(A_R) \left( \int_z Re\big(a_M(z)G(t,z)\big)dz \right) dt$$

$$+ \int_{pT_{US}}^{pT_{US}+T_{US}/4} 4Im(A_R) \left( \int_z Im\big(a_M(z)G(t,z)\big)dz \right) dt$$

$$\left. - \int_{pT_{US}+T_{US}/2}^{pT_{US}+3T_{US}/4} 4Im(A_R) \left( \int_z Im\big(a_M(z)G(t,z)\big)dz \right) dt \right)$$

Les termes Q et I peuvent être ré-exprimés comme suit :

[Math 15]

$$Q = \sum_{p=1}^{N} \Pi(p) \left( \int_{pT_{US}+T_{US}/4}^{pT_{US}+T_{US}/2} 4Re(A_R) \left( \int_z \left( Re(a_M(z))Re(G(t,z)) \right. \right. \right.$$

$$\left. - Im(a_M(z))Im(G(t,z)) \right) dz \right) dt$$

$$- \int_{pT_{US}+3T_{US}/4}^{pT_{US}+T_{US}} 4Re(A_R) \left( \int_z \left( Re(a_M(z))Re(G(t,z)) \right. \right.$$

$$\left. - Im(a_M(z))Im(G(t,z)) \right) dz \right) dt$$

$$+ \int_{pT_{US}+T_{US}/4}^{pT_{US}+T_{US}/2} 4Im(A_R) \left( \int_z \left( Re(a_M(z))Im(G(t,z)) \right. \right.$$

$$\left. + Im(a_M(z))Re(G(t,z)) \right) dz \right) dt$$

$$- \int_{pT_{US}+3T_{US}/4}^{pT_{US}+T_{US}} 4Im(A_R) \left( \int_z \left( Re(a_M(z))Im(G(t,z)) \right. \right.$$

$$\left. \left. \left. + Im(a_M(z))Re(G(t,z)) \right) dz \right) dt \right)$$

Et :

[Math 16]

$$I = \sum_{p=1}^{N} \Pi(p) \Bigg( \int_{pT_{US}}^{pT_{US}+T_{US}/4} 4Re(A_R) \bigg( \int_z \Big( Re\big(a_M(z)\big)Re\big(G(t,z)\big)$$

$$- Im\big(a_M(z)\big)Im\big(G(t,z)\big) \Big) dz \bigg) dt$$

$$- \int_{pT_{US}+T_{US}/2}^{pT_{US}+3T_{US}/4} 4Re(A_R) \bigg( \int_z \Big( Re\big(a_M(z)\big)Re\big(G(t,z)\big)$$

$$- Im\big(a_M(z)\big)Im\big(G(t,z)\big) \Big) dz \bigg) dt$$

$$+ \int_{pT_{US}}^{pT_{US}+T_{US}/4} 4Im(A_R) \bigg( \int_z \Big( Re\big(a_M(z)\big)Im\big(G(t,z)\big)$$

$$+ Im\big(a_M(z)\big)Re\big(G(t,z)\big) \Big) dz \bigg) dt$$

$$- \int_{pT_{US}+T_{US}/2}^{pT_{US}+3T_{US}/4} 4Im(A_R) \bigg( \int_z \Big( Re\big(a_M(z)\big)Im\big(G(t,z)\big)$$

$$+ Im\big(a_M(z)\big)Re\big(G(t,z)\big) \Big) dz \bigg) dt \Bigg)$$

Soit :

[Math 17]

$$Q = \int_z Re(a_M(z)) \left( 4Re(A_R) \left( \sum_{p=1}^{N} \Pi(p) \left( \int_{pT_{US}+T_{US}/4}^{pT_{US}+T_{US}/2} Re(G(t,z))dt - \int_{pT_{US}+3T_{US}/4}^{pT_{US}+T_{US}} Re(G(t,z))dt \right) \right) \right.$$

$$+ 4Im(A_R) \left( \sum_{p=1}^{N} \Pi(p) \left( \int_{pT_{US}+T_{US}/4}^{pT_{US}+T_{US}/2} Im(G(t,z))dt \right. \right.$$

$$\left. \left. \left. - \int_{pT_{US}+3T_{US}/4}^{pT_{US}+T_{US}} Im(G(t,z))dt \right) \right) \right) dz$$

$$- \int_z Im(a_M(z)) \left( 4Re(A_R) \left( \sum_{p=1}^{N} \Pi(p) \left( \int_{pT_{US}+T_{US}/4}^{pT_{US}+T_{US}/2} Im(G(t,z))dt \right. \right. \right.$$

$$\left. \left. - \int_{pT_{US}+3T_{US}/4}^{pT_{US}+T_{US}} Im(G(t,z))dt \right) \right)$$

$$- 4Im(A_R) \left( \sum_{p=1}^{N} \Pi(p) \left( \int_{pT_{US}+T_{US}/4}^{pT_{US}+T_{US}/2} Re(G(t,z))dt \right. \right.$$

$$\left. \left. \left. \left. - \int_{pT_{US}+3T_{US}/4}^{pT_{US}+T_{US}} Re(G(t,z))dt \right) \right) \right) \right) dz$$

Et :

[Math 18]

$$I = \int_z Re\big(a_M(z)\big)\Bigg(4Re(A_R)\Bigg(\sum_{p=1}^{N}\Pi(p)\Bigg(\int_{pT_{US}}^{pT_{US}+T_{US}/4}Re\big(G(t,z)\big)dt$$

$$-\int_{pT_{US}+T_{US}/2}^{pT_{US}+3T_{US}/4}Re\big(G(t,z)\big)dt\Bigg)\Bigg)$$

$$+4Im(A_R)\Bigg(\sum_{p=1}^{N}\Pi(p)\Bigg(\int_{pT_{US}}^{pT_{US}+T_{US}/4}Im\big(G(t,z)\big)dt$$

$$-\int_{pT_{US}+T_{US}/2}^{pT_{US}+3T_{US}/4}Im\big(G(t,z)\big)dt\Bigg)\Bigg)\Bigg)dz$$

$$-\int_z Im\big(a_M(z)\big)\Bigg(4Re(A_R)\Bigg(\sum_{p=1}^{N}\Pi(p)\Bigg(\int_{pT_{US}}^{pT_{US}+T_{US}/4}Im\big(G(t,z)\big)dt$$

$$-\int_{pT_{US}+T_{US}/2}^{pT_{US}+3T_{US}/4}Im\big(G(t,z)\big)dt\Bigg)\Bigg)$$

$$-4Im(A_R)\Bigg(\sum_{p=1}^{N}\Pi(p)\Bigg(\int_{pT_{US}}^{pT_{US}+T_{US}/4}Re\big(G(t,z)\big)dt$$

$$-\int_{pT_{US}+T_{US}/2}^{pT_{US}+3T_{US}/4}Re\big(G(t,z)\big)dt\Bigg)\Bigg)\Bigg)dz$$

En désignant par $Q_j$, respectivement $I_j$, le terme Q, respectivement I, mesuré par le pixel à chacune des M mesures de la phase d'acquisition du profil d'absorption, et en affectant d'un indice j tous les termes variant d'une mesure à l'autre, avec j entier allant de 1 à M, on peut réécrire, pour chaque mesure, les équations comme suit :

[Math 19]

$$Q_j = \int_z Re\big(a_M(z)\big)C_j(z)\,dz - \int_z Im\big(a_M(z)\big)D_j(z)dz$$

Et :

[Math 20]

$$I_j = \int_z Re\big(a_M(z)\big) E_j(z)\, dz - \int_z Im\big(a_M(z)\big) F_j(z) dz$$

Avec, sachant que Im(G(t,z))=0 :

[Math 21]

$$C_j(z) = 4Re(A_R) \sum_{p=1}^{N} \Pi_j(p) \left( \int_{pT_{US}+T_{US}/4}^{pT_{US}+T_{US}/2} cos\Big(\omega_{US}(t - z/v_{US}) \right.$$
$$+ \pi\Phi_j\big(t - z/v_{US} + \delta_j\big)\Big) dt$$
$$\left. - \int_{pT_{US}+3T_{US}/4}^{pT_{US}+T_{US}} cos\Big(\omega_{US}(t - z/v_{US}) + \pi\Phi_j\big(t - z/v_{US} + \delta_j\big)\Big) dt \right)$$

[Math 22]

$$D_j(z) = -4Im(A_R) \left( \sum_{p=1}^{N} \Pi_j(p) \left( \int_{pT_{US}+T_{US}/4}^{pT_{US}+T_{US}/2} cos\Big(\omega_{US}(t - z/v_{US}) \right. \right.$$
$$+ \pi\Phi_j\big(t - z/v_{US} + \delta_j\big)\Big) dt$$
$$\left. \left. - \int_{pT_{US}+3T_{US}/4}^{pT_{US}+T_{US}} cos\Big(\omega_{US}(t - z/v_{US}) + \pi\Phi_j\big(t - z/v_{US} + \delta_j\big)\Big) dt \right) \right)$$

[Math 23]

$$E_j(z) = 4Re(A_R) \left( \sum_{p=1}^{N} \Pi_j(p) \left( \int_{pT_{US}}^{pT_{US}+T_{US}/4} cos\Big(\omega_{US}(t - z/v_{US}) \right. \right.$$
$$+ \pi\Phi_j\big(t - z/v_{US} + \delta_j\big)\Big) dt$$
$$\left. \left. - \int_{pT_{US}+T_{US}/2}^{pT_{US}+3T_{US}/4} cos\Big(\omega_{US}(t - z/v_{US}) + \pi\Phi_j\big(t - z/v_{US} + \delta_j\big)\Big) dt \right) \right)$$

[Math 24]

$$F_j(z) = -4Im(A_R)\left(\sum_{p=1}^{N}\Pi_j(p)\left(\int_{pT_{US}}^{pT_{US}+T_{US}/4} cos\left(\omega_{US}(t-z/v_{US})\right.\right.\right.$$

$$\left.\left.+\pi\Phi_j\left(t-z/v_{US}+\delta_j\right)\right)dt$$

$$\left.\left.-\int_{pT_{US}+T_{US}/2}^{pT_{US}+3T_{US}/4} cos\left(\omega_{US}(t-z/v_{US})+\pi\Phi_j\left(t-z/v_{US}+\delta_j\right)\right)dt\right)\right)$$

L'ensemble des mesures obtenues peut être écrit sous la forme d'un vecteur Y de M nombres imaginaires $Y_j$, avec $Y_j = I_j + iQ_j$, et :

[Math 25]

$$Q_j = \sum_{k=1}^{L}\left(Re(a_M(k.\Delta z))\int_{(k-1).\Delta z}^{k.\Delta z}C_j(z)dz - Im(a_M(k.\Delta z))\int_{(k-1).\Delta z}^{k.\Delta z}D_j(z)dz\right)$$

[Math 26]

$$I_j = \sum_{k=1}^{L}\left(Re(a_M(k.\Delta z))\int_{(k-1).\Delta z}^{k.\Delta z}E_j(z)dz - Im(a_M(k.\Delta z))\int_{(k-1).\Delta z}^{k.\Delta z}F_j(z)dz\right)$$

Où $\Delta z$ représente une fraction de la longueur d'onde $\lambda_{US} = T_{US}*v_{US}$ du signal acoustique de pulsation $\omega_{US}$, définissant la résolution axiale de mesure souhaitée ($L*\Delta z$ correspondant à l'épaisseur du milieu analysé, c'est-à-dire la longueur de la colonne de marquage dont on souhaite acquérir le profil d'absorption). L'absorption du milieu dans un tronçon de longueur $\Delta z$ de la colonne de marquage est considérée constante sur la longueur $\Delta z$ du tronçon.

Dans cet exemple, pour construire la matrice de passage du système, on réécrit les termes I et Q en considérant, pour chaque position z le long de la colonne de marquage, une contribution de référence prédéterminée $a_{Mref}(z)$ à la partie marquée du faisceau objet reçue par le capteur, permettant par exemple de modéliser l'efficacité de marquage acoustique le long de la colonne (par exemple pour tenir compte d'une éventuelle non uniformité de la densité de puissance acoustique le long de la colonne). Le terme $a_{Mref}$ est un vecteur prédéterminé de L valeurs, défini en fonction des propriétés du système d'imagerie, et notamment des propriétés du transducteur utilisé pour générer l'onde acoustique de marquage. On peut alors écrire, pour chacune des M mesures :

[Math 27]

$$Q_{j\,Ref} = \sum_{k=1}^{L}\left(Re\left(a_{M\,Ref}(k.\Delta z)\right)\int_{(k-1).\Delta z}^{k.\Delta z}C_j(z)dz\right.$$

$$\left.- Im\left(a_{M\,Ref}(k.\Delta z)\right)\int_{(k-1).\Delta z}^{k.\Delta z}D_j(z)dz\right)$$

Et :

[Math 28]

$$I_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$\left. - Im\left(a_{M\,Ref}(k.\Delta z)\right) \int_{(k-1).\Delta z}^{k.\Delta z} F_j(z)dz \right)$$

Puis :

[Math 29]

$$Y_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$\left. - Im\left(a_{M\,Ref}(k.\Delta z)\right) \int_{(k-1).\Delta z}^{k.\Delta z} F_j(z)dz \right)$$

$$+ i \sum_{k=1}^{L} \left( Re\left(a_{M\,Ref}(k.\Delta z)\right) \int_{(k-1).\Delta z}^{k.\Delta z} C_j(z)dz \right.$$

$$\left. - Im\left(a_{M\,Ref}(k.\Delta z)\right) \int_{(k-1).\Delta z}^{k.\Delta z} D_j(z)dz \right)$$

[Math 30]

$$Y_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$- Im\left(a_{M\,Ref}(k.\Delta z)\right) \int_{(k-1).\Delta z}^{k.\Delta z} F_j(z)dz$$

$$+ i \left( Re\left(a_{M\,Ref}(k.\Delta z)\right) \int_{(k-1).\Delta z}^{k.\Delta z} C_j(z)dz \right.$$

$$\left.\left. - Im\left(a_{M\,Ref}(k.\Delta z)\right) \int_{(k-1).\Delta z}^{k.\Delta z} D_j(z)dz \right) \right)$$

[Math 31]

$$Y_{j\,Ref} = \sum_{k=1}^{L} \left( Re\left( a_{M\,Ref}(k.\Delta z) \right) \int_{(k-1).\Delta z}^{k.\Delta z} \left( E_j(z) + iC_j(z) \right) dz \right.$$

$$\left. - Im\left( a_{M\,Ref}(k.\Delta z) \right) \int_{(k-1).\Delta z}^{k.\Delta z} \left( F_j(z) + iD_j(z) \right) dz \right)$$

[Math 32]

$$Y_{j\,Ref} = \sum_{k=1}^{L} \left( 4Re(A_R)Re\left( a_{M\,Ref}(k.\Delta z) \right) \int_{(k-1).\Delta z}^{k.\Delta z} \left( S13_j(z) + iS24_j(z) \right) dz \right.$$

$$\left. + 4Im(A_R)Im\left( a_{M\,Ref}(k.\Delta z) \right) \int_{(k-1).\Delta z}^{k.\Delta z} \left( S13_j(z) + iS24_j(z) \right) dz \right)$$

Avec :

[Math 33]

$$S13_j(z) = \sum_{p=1}^{N} \Pi_j(p) \left( \int_{pT_{US}}^{pT_{US}+T_{US}/4} cos\left( \omega_{US}(t - z/v_{US}) + \pi\Phi_j(t - z/v_{US} + \delta_j) \right) dt \right.$$

$$\left. - \int_{pT_{US}+T_{US}/2}^{pT_{US}+3T_{US}/4} cos\left( \omega_{US}(t - z/v_{US}) + \pi\Phi_j(t - z/v_{US} + \delta_j) \right) dt \right)$$

Et :

[Math 34]

$$S24_j(z) = \sum_{p=1}^{N} \Pi_j(p) \left( \int_{pT_{US}+T_{US}/4}^{pT_{US}+T_{US}/2} cos\left( \omega_{US}(t - z/v_{US}) + \pi\Phi_j(t - z/v_{US} + \delta_j) \right) dt \right.$$

$$\left. - \int_{pT_{US}+3T_{US}/4}^{pT_{US}+T_{US}} cos\left( \omega_{US}(t - z/v_{US}) + \pi\Phi_j(t - z/v_{US} + \delta_j) \right) dt \right)$$

On réécrit alors :

[Math 35]

$$Y_{j\,Ref} = \sum_{k=1}^{L} \Bigg( \Big( 4Re(A_R)Re\Big(a_{M\,Ref}(k.\Delta z)\Big)$$

$$+ 4Im(A_R)Im\Big(a_{M\,Ref}(k.\Delta z)\Big)\Big) \int_{(k-1).\Delta z}^{k.\Delta z} \Big(S13_j(z) + iS24_j(z)\Big)dz \Bigg)$$

En ayant identifié la relation entre les mesures et le profil d'absorption selon l'équation Math 35, on peut établir la relation matricielle suivante :

[Math 36]

$$Y = \mathcal{A}_{Re}Re(a_M) + \mathcal{A}_{Im}Im(a_M)$$

Où $A_{Re}$ et $A_{Im}$ sont deux matrices ayant chacune M rangées et L colonnes, et $a_M$ est un vecteur de L rangées et une colonne dont le module correspond au profil d'absorption recherché.

Le terme $A_{Re\,j,k}$ désigne la valeur de la rangée de rang j et de la colonne de rang k dans la matrice $A_{Re}$, et le terme $A_{Im\,j,k}$ désigne la valeur de la rangée de rang j et de la colonne de rang k dans la matrice $A_{Im}$.

Les valeurs $A_{Re\,j,k}$ et $A_{Im\,j,k}$ des matrices $A_{Re}$ et $A_{Im}$ peuvent s'exprimer comme suit :

[Math 37]

$$\mathcal{A}_{Re\,j,k} = 4Re(A_R)Re\Big(a_{M\,Ref}(k.\Delta z)\Big) \int_{(k-1).\Delta z}^{k.\Delta z} \Big(S13_j(z) + iS24_j(z)\Big)dz$$

Et :

[Math 38]

$$\mathcal{A}_{Im\,j,k} = 4Im(A_R)Im\Big(a_{M\,Ref}(k.\Delta z)\Big) \int_{(k-1).\Delta z}^{k.\Delta z} \Big(S13_j(z) + iS24_j(z)\Big)dz$$

Les matrices $A_{Re\,j,k}$ et $A_{Im\,j,k}$ sont les matrices de passage du système. En considérant M mesures (j varie de 1 à M), ces matrices sont chacune constituées de M rangées et L colonnes, L correspondant au nombre de points du profil d'absorption. A la différence de la solution présentée en relation avec les figures 3 et 4, dans le mode de réalisation de la figure 4, le nombre M de mesures réalisées peut être inférieur au nombre L de points du profil d'absorption que l'on souhaite reconstruire.

Ainsi, on dispose d'un modèle tel que :

[Math 39]

$$Y = Fct(a_M) + \varepsilon$$

où Y est un vecteur de M nombres imaginaires $Y_j$ correspondant respectivement aux M mesures effectuées par le capteur, Fct est une fonction du profil d'absorption $a_M$, et $\varepsilon$ est un terme d'erreur que l'on va chercher à minimiser.

On recherche donc une estimée du profil d'absorption $a_M$, notée comme suit :

[Math 40]

$$\widehat{a_M}$$

Pour cela, on cherche à résoudre le problème de minimisation d'erreur suivant :

[Math 41]

$$\underset{\widehat{a_M}}{\mathrm{argmin}}(\|Y - Fct(\widehat{a_M})\|_2^2)$$

Ce problème présentant une infinité de solutions, on ajoute un terme de régularisation permettant de le résoudre.

Connaissant les matrices de passage $A_{Re\,j,k}$ et $A_{Im\,j,k}$, la reconstruction du profil d'absorption défini par le vecteur $a_M$ peut être réalisée en minimisant la fonction de coût suivante :

[Math 42]

$$\underset{\widehat{a_M}}{\mathrm{argmin}} \left( \left\| \left( \mathcal{A}_{Re} Re(\widehat{a_M}) + \mathcal{A}_{Im} Im(\widehat{a_M}) \right) - Y \right\|_2 + \lambda \| \psi | \widehat{a_M} | \|_1 \right)$$

où $\psi$ est une fonction définissant une contrainte de reconstruction sur le module du vecteur $a_M$, correspondant à des propriétés supposées du signal à reconstruire. Le coefficient $\lambda$ permet de pondérer la contribution de la contrainte de reconstruction dans la fonction de coût globale. Dans cet exemple, la contrainte est définie à partir de la norme 1 de la fonction $\psi$, multipliée par l'estimée du profil d'absorption $a_M$. A titre d'exemple, la fonction $\psi$ est un banc de filtres réalisé à partir d'un opérateur différentiel selon l'axe de propagation de l'onde acoustique, dupliqué à différentes échelles pour chacune des positions z. Plus généralement, toute autre méthode de synthèse de la contrainte de reconstruction, permettant de tenir compte de propriétés supposées du signal recherché, peut être utilisée.

A partir de cette équation, on reconstruit un vecteur $a_M$ dont le module correspond au profil d'absorption recherché.

La résolution du problème de minimisation défini par l'équation Math 42 est par exemple mise en oeuvre par un circuit électronique de traitement (non visible sur la figure), comprenant par exemple un microprocesseur. Le circuit de traitement peut être intégré au capteur 109, ou extérieur au capteur 109.

On notera que, dans le mode de réalisation de la figure 5, la matrice de passage $A_{Re\,j,k}$ peut s'exprimer comme suit :

[Math 43]

$$\mathcal{A}_{Re\,j,k} = \int_{(k-1).\Delta z}^{k.\Delta z} \left( 4Re(A_R)Re\left(a_{M\,Ref}(k.\Delta z)\right) \sum_{p=1}^{N} \Pi_j(p) \left( \left( \int_{pT_{US}}^{pT_{US}+T_{US}/4} cos\left(\omega_{US}(t \right. \right. \right. \right.$$

$$\left. - z/v_{US}) + \pi\Phi_j\left(t - z/v_{US} + \delta_j\right)\right) dt$$

$$\left. - \int_{pT_{US}+T_{US}/2}^{pT_{US}+3T_{US}/4} cos\left(\omega_{US}(t - z/v_{US}) + \pi\Phi_j\left(t - z/v_{US} + \delta_j\right)\right) dt \right)$$

$$\left. + i\left( \int_{pT_{US}+T_{US}/4}^{pT_{US}+T_{US}/2} cos\left(\omega_{US}(t - z/v_{US}) + \pi\Phi_j\left(t - z/v_{US} + \delta_j\right)\right) dt \right. \right.$$

$$\left. \left. \left. \left. - \int_{pT_{US}+3T_{US}/4}^{pT_{US}+T_{US}} cos\left(\omega_{US}(t - z/v_{US}) + \pi\Phi_j\left(t - z/v_{US} + \delta_j\right)\right) dt \right) \right) \right) dz$$

Soit :

[Math 44]

$$\mathcal{A}_{Re\,j,k} = \int_{(k-1).\Delta z}^{k.\Delta z} \left( 4Re(A_R)Re\left(a_{M\,Ref}(k.\Delta z)\right) \sum_{p=1}^{N} \Pi_j(p)\,\Gamma_j(p,z) \right) dz$$

Avec :

[Math 45]

$$\Gamma_j(p,z) = \left( \left( \int_{pT_{US}}^{pT_{US}+T_{US}/4} cos\left(\omega_{US}(t - z/v_{US}) + \pi\Phi_j\left(t - z/v_{US} + \delta_j\right)\right) dt \right. \right.$$

$$\left. - \int_{pT_{US}+T_{US}/2}^{pT_{US}+3T_{US}/4} cos\left(\omega_{US}(t - z/v_{US}) + \pi\Phi_j\left(t - z/v_{US} + \delta_j\right)\right) dt \right)$$

$$\left. + i\left( \int_{pT_{US}+T_{US}/4}^{pT_{US}+T_{US}/2} cos\left(\omega_{US}(t - z/v_{US}) + \pi\Phi_j\left(t - z/v_{US} + \delta_j\right)\right) dt \right. \right.$$

$$\left. \left. \left. - \int_{pT_{US}+3T_{US}/4}^{pT_{US}+T_{US}} cos\left(\omega_{US}(t - z/v_{US}) + \pi\Phi_j\left(t - z/v_{US} + \delta_j\right)\right) dt \right) \right)$$

La combinaison de $\Pi$ et de $\Gamma$ donne une loi de Rademacher, similaire à une loi de Bernouilli, dont la somme tend, selon le théorème de Moivre Laplace, vers une variable aléatoire de distribution gaussienne. Les coefficients de la matrice de passage $A_{Re\,j,k}$ s'apparentent asymptotiquement à des tirages d'une distribution gaussienne, permettant une reconstruction de type acquisition compressive.

Un raisonnement similaire s'applique concernant la matrice de passage $A_{Im\,j,k}$.

Bien que l'on ait décrit ci-dessus un exemple de fonctionnement basé sur une démodulation à K=4 phases du signal lumineux modulé, le mode de réalisation de la figure 5 ne se limite pas à ce cas particulier. A titre de variante, la démodulation peut être réalisée sur tout autre nombre K de phases supérieur ou égal à 2. L'homme du métier saura adapter en conséquence les circuits des pixels, notamment pour acquérir un nombre d'échantillons différent de quatre à chaque période de modulation $T_{US}$ de la phase d'acquisition $T_{acq}$. Les formules de reconstruction des parties réelle et imaginaire du champ complexe du faisceau objet pourront être adaptées en conséquence.

De plus, bien que, dans l'exemple décrit ci-dessus en relation avec la figure 5, on ait considéré une phase de l'onde acoustique de marquage nulle à l'origine, les modes de réalisation décrits ne se limitent pas à ce cas particulier. A titre de variante, les calculs décrits ci-dessus peuvent être repris en ajoutant une phase $\varphi$ non nulle telle que :

[Math 46]

$$E_s(t) = A_D e^{i\omega_L t} + \int_z a_M(z) e^{-i\omega_{US} z/v_{US}} e^{i((\omega_L+\omega_{US})t + \Phi(t-z/v_{US}+\delta)+\varphi)} dz$$

$$+ \int_z a_M(z) e^{i\omega_{US} z/v_{US}} e^{i((\omega_L-\omega_{US})t - \Phi(t-z/v_{US}+\delta)+\varphi)} dz$$

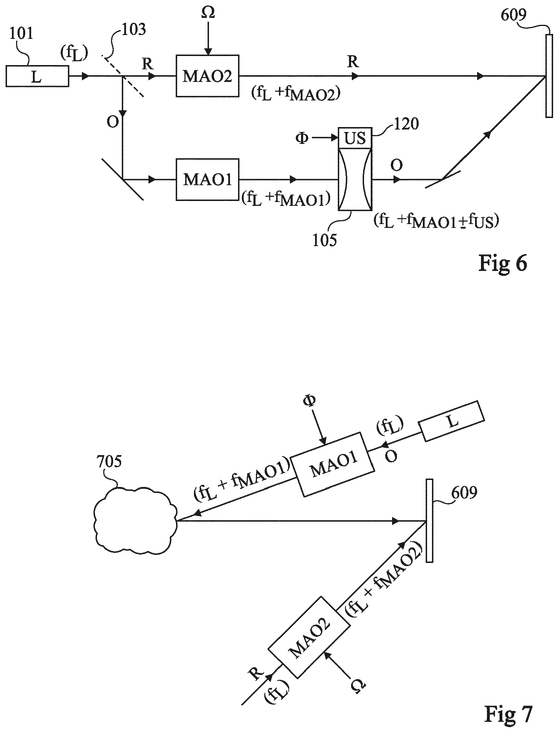

La figure 6 représente de façon schématique un exemple d'un système d'imagerie acousto-optique selon un deuxième mode de réalisation.

Le système de la figure 6 comprend, comme dans les exemples précédents, une source lumineuse 101 (L) adaptée à générer un faisceau lumineux cohérent de fréquence $f_L$, et un séparateur 103 permettant de diviser le faisceau produit par la source 101 en un faisceau objet O et un faisceau de référence R. Dans le système de la figure 6, en sortie du séparateur 103, le faisceau objet O est modulé par un premier modulateur acousto-optique MAO1 décalant sa fréquence d'une valeur $f_{MAO1}$, et le faisceau de référence R est modulé par un deuxième modulateur acousto-optique MAO2 décalant sa fréquence d'une valeur $f_{MAO2}$ différente de la valeur $f_{MO1}$. Le système est agencé de façon que le faisceau objet, de fréquence $f_L+f_{MAO1}$, illumine l'objet ou échantillon 105 à analyser, et que le faisceau de référence, de fréquence $f_L+f_{MAO2}$, ne passe pas par l'échantillon 105.

Comme dans les exemples précédents, le faisceau de référence et le faisceau objet réfléchi ou transmis par l'objet 105 sont ensuite projetés sur un capteur d'images 609, de manière à produire une figure d'interférence dans le plan d'acquisition du capteur.

De plus, comme dans les exemples précédents, une partie de l'échantillon 105 est excitée par une onde ultrasonore focalisée de fréquence $F_{US}$. Il en résulte que la fréquence des rayons du faisceau objet passant par la partie excitée de l'échantillon est décalée à la valeur $f_L+f_{MAO1}+F_{US}$ ou $f_L+f_{MAO1}-F_{US}$, alors que la fréquence des rayons du faisceau objet passant par les parties non-excitées de l'échantillon reste à la valeur $f_L+f_{MAO1}$. Comme précédemment, on cherche à mesurer l'énergie portée par les photons marqués par l'onde acoustique de fréquence $F_{US}$, pour en déduire des informations quant à l'absorption lumineuse dans la zone de marquage.

En fonctionnement, le capteur 609 voit une figure d'interférences présentant une fréquence de battement $f_B$ égale à la valeur absolue de la différence $f_{MAO1}+F_{US}-f_{MAO2}$.

A la différence du capteur 109 décrit précédemment, comprenant un dispositif de démodulation électronique, par exemple sous la forme d'un circuit d'intégration à K capacités commutées par pixel, cadencé de façon synchrone à une fréquence égale à K fois la fréquence $F_{US}$ de l'onde acoustique de marquage, dans le mode de réalisation de la figure 6 le capteur 609 est un capteur standard, comportant par exemple un circuit d'intégration à capacité unique par pixel. Dans cet exemple, la fréquence $F_C$ d'acquisition des images par le capteur 609 est égale à K fois la fréquence de battement $f_B$ du système. Le montage à deux modulateurs acousto-optiques MAO1 et MAO2 de la figure 6 permet d'utiliser un capteur 609 présentant une fréquence d'acquisition $F_C$ relativement basse, par exemple comprise entre 20 Hz et 20 kHz, ce qui correspond aux fréquences d'acquisition typiques des capteurs d'images usuels.

Les modulateurs acousto-optiques usuels peuvent typiquement décaler la fréquence d'un faisceau lumineux d'une valeur de l'ordre de quelques MHz à quelques dizaines de MHz, par exemple d'une valeur comprise entre 5 et 100 MHz. L'onde ultrasonore de marquage appliquée à l'échantillon 105 provoque quant à elle un décalage de fréquence de la lumière passant par la partie excitée de l'objet d'une valeur comprise, par exemple, entre 1 et 15 MHz. Les modulateurs MAO1 et MAO2 sont choisis de façon à obtenir une fréquence de battement $f_B$ compatible avec l'utilisation d'un capteur basse fréquence, par exemple tels que la fréquence $F_C=K*f_B$ soit comprise entre 20 Hz et 20 kHz.

A titre d'exemple numérique illustratif et non limitatif, dans le système de la figure 6, le capteur 609 peut avoir une fréquence d'acquisition $F_C$ de 8 kHz, et les décalages fréquentiels $f_{MAO1}$, $f_{MAO2}$ et $F_{US}$ introduits par les modulateurs MAO1 et MAO2 et par l'onde ultrasonore d'excitation de l'échantillon 105 peuvent être respectivement de 77,001 MHz, 79,999 MHz, et 3 MHz, pour obtenir une fréquence de battement $f_B = F_{MAO1} + F_{US} - f_{MAO2} = 2$ kHz $= F_C/4$ (en considérant le cas K=4).

Comme dans le mode de réalisation de la figure 5, on prévoit de reconstruire un profil d'absorption de la colonne de marquage, de résolution axiale L, à partir d'une série de M mesures, avec L et M entiers supérieurs ou égaux à 2.

Lors de chaque mesure, une première séquence de sauts de phase Φ aléatoire ou pseudo-aléatoire est appliquée au signal acoustique de marquage, de façon identique ou similaire à ce qui a été décrit dans l'exemple de la figure 5.

Lors de la phase d'acquisition, de durée $T_{acq} = T_B = 1/f_B$, on acquiert successivement K images au moyen du capteur 609. Pour chacune des M mesures, on obtient donc, pour chaque pixel, un jeu de K valeurs correspondant aux quatre phases de la période de battement $T_B$. Par souci de simplification, on considérera ci-après le cas K=4, et on désignera par I0, I1, I2 et I3 les quatre échantillons acquis par un pixel pendant une même phase d'acquisition $T_{acq}$ (correspondant aux valeurs du pixel dans les 4 images successivement acquises pendant la phase $T_{acq}$).

Comme dans le mode de réalisation de la figure 5, on prévoit, à chaque mesure, d'appliquer au signal mesuré une séquence de démodulation Ω, sous la forme d'une séquence de sauts de phase aléatoire ou pseudo-aléatoire non corrélée à la séquence Φ.

Le mode de réalisation de la figure 6 diffère du mode de réalisation de la figure 5 en ce que, dans le mode de réalisation de la figure 6, la séquence de démodulation Ω est appliquée non pas dans le domaine électronique, par un dispositif de démodulation intégré au capteur, mais dans le domaine optique, par l'intermédiaire du modulateur acousto-optique MAO2 placé sur le bras de référence R. Autrement dit, dans cet exemple, la séquence de démodulation Ω est une séquence aléatoire ou pseudo-aléatoire appliquée directement au faisceau de référence R, avant interférence du faisceau R avec le faisceau objet O.

Le signal s(t) mesuré au niveau d'un pixel du capteur 609 à l'issue de l'interférence entre le bras objet et le bras de référence est donné par l'équation suivante :

[Math 47]

$$s(t) = |E_r(t) + E_s(t)|^2$$

Avec :

[Math 48]

$$E_r(t) = A_R e^{i(\omega_R t + \Omega(t - \delta))}$$

Et :

[Math 49]

$$E_s(t) = A_D e^{i\omega_o t} + \int_Z a_M(z) e^{i(\omega_o t + \omega_{US}(t - z/v_{US}) + \Phi(t - z/v_{US}))} dz$$

$$+ \int_Z a_M(z) e^{i(\omega_o t - \omega_{US}(t - z/v_{US}) - \Phi(t - z/v_{US}))} dz$$

Soit :

[Math 50]

$$E_s(t) = A_D e^{i\omega_O t} + \int_Z a_{M+}(z) e^{i((\omega_O + \omega_{US})t + \Phi(t - z/v_{US}))} dz$$

$$+ \int_Z a_{M-}(z) e^{i((\omega_O - \omega_{US})t - \Phi(t - z/v_{US}))} dz$$

Où :

[Math 51]

$$a_{M+}(z) = a_M(z) e^{-i\omega_{US} z/v_{US}}$$

[Math 52]

$$a_{M-}(z) = a_M(z) e^{i\omega_{US} z/v_{US}}$$

Et où :

[Math 53]

$$\omega_R = 2\pi(f_L + f_{MAO2})$$

[Math 54]

$$\omega_O = 2\pi(f_L + f_{MAO1})$$

[Math 55]

$$\omega_R - \omega_O = \omega_{US} - \omega_{CAM}$$

[Math 56]

$$\omega_{CAM} = 2\pi F_{CAM}$$

Où $F_{CAM}$ désigne la fréquence d'acquisition du système, telle que $F_{CAM} = 1/T_{acq} = F_C/4$.

L'équation Math 50 peut être réécrite comme suit :

[Math 57]

$$E_s(t) = A_D e^{i\omega_O t} + \int_Z a_{M+}(z) e^{i((\omega_R + \omega_{CAM})t + \Phi(t - z/v_{US}))} dz$$

$$+ \int_Z a_{M-}(z) e^{i((\omega_R - 2\omega_{US} + \omega_{CAM})t - \Phi(t - z/v_{US}))} dz$$

D'où :

[Math 68]

$$s(t) = \left( \left| e^{i(\omega_R t + \Omega(t-\delta))} \right| \cdot \left| A_R + A_D e^{i((\omega_O - \omega_R)t - \Omega(t-\delta))} \right. \right.$$

$$+ \int_Z a_{M+}(z) e^{i((\omega_R + \omega_{CAM})t + \Phi(t - z/v_{US}) - \omega_R t - \Omega(t-\delta))} dz$$

$$\left. \left. + \int_Z a_{M-}(z) e^{i((\omega_R - 2\omega_{US} + \omega_{CAM})t - \Phi(t - z/v_{US}) - \omega_R t - \Omega(t-\delta))} dz \right| \right)^2$$

[Math 59]

$$s(t) = \left| A_R + A_D e^{i((\omega_O - \omega_R)t - \Omega(t-\delta))} \right.$$

$$+ \int_Z a_M(z) e^{i(\omega_{CAM}t - \omega_{US}z/v_{US} + \Phi(t - z/v_{US}) - \Omega(t-\delta))} dz$$

$$\left. + \int_Z a_M(z) e^{i((\omega_{CAM} - 2\omega_{US})t + \omega_{US}z/v_{US} - \Phi(t - z/v_{US}) - \Omega(t-\delta))} dz \right|^2$$

Au niveau du capteur 609, on acquiert successivement K=4 images avec une durée d'acquisition $T_{CAM}/4$ par image (avec $T_{CAM}=1/F_{CAM}$). Les voies I et Q peuvent être exprimées comme suit :

[Math 60]

$$I = \int_0^{T_{CAM}/4} s(t)dt - \int_{T_{CAM}/2}^{3T_{CAM}/4} s(t)dt$$

[Math 61]

$$Q = \int_{T_{CAM}/4}^{T_{CAM}/2} s(t)dt - \int_{3T_{CAM}/4}^{T_{CAM}} s(t)dt$$

On définit des fonctions F et G telles que :

[Math 62]

$$F(z,t) = e^{i(\omega_{CAM}t - \omega_{US}z/v_{US} + \Phi(t - z/v_{US}) - \Omega(t-\delta))}$$

[Math 63]

$$G(z,t) = e^{i((\omega_{CAM} - 2\omega_{US})t + \omega_{US}z/v_{US} - \Phi(t - z/v_{US}) - \Omega(t-\delta))}$$

On a alors :

[Math 64]

$$I = \int_0^{T_{CAM}/4} \left(\left(Re\left(A_R + A_D e^{i((\omega_O - \omega_R)t - \Omega(t-\delta))} + \int_z a_M(z)\big(F(z,t) + G(z,t)\big)dz\right)\right)^2\right.$$

$$+ \left(Im\left(A_R + A_D e^{i((\omega_O - \omega_R)t - \Omega(t-\delta))}\right.\right.$$

$$\left.\left.\left.+ \int_z a_M(z)\big(F(z,t) + G(z,t)\big)dz\right)\right)^2\right)dt$$

$$- \int_{T_{CAM}/2}^{3T_{CAM}/4} \left(\left(Re\left(A_R + A_D e^{i((\omega_O - \omega_R)t - \Omega(t-\delta))}\right.\right.\right.$$

$$\left.\left.+ \int_z a_M(z)\big(F(z,t) + G(z,t)\big)dz\right)\right)^2$$

$$+ \left(Im\left(A_R + A_D e^{i((\omega_O - \omega_R)t - \Omega(t-\delta))}\right.\right.$$

$$\left.\left.\left.\left.+ \int_z a_M(z)\big(F(z,t) + G(z,t)\big)dz\right)\right)^2\right)\right)dt$$

[Math 65]

$$Q = \int_{T_{CAM}/4}^{T_{CAM}/2} \left( \left( Re \left( A_R + A_D e^{i((\omega_O - \omega_R)t - \Omega(t-\delta))} \right. \right. \right.$$

$$\left. \left. + \int_Z a_M(z)\big(F(z,t) + G(z,t)\big)dz \right) \right)^2$$

$$+ \left( Im \left( A_R + A_D e^{i((\omega_O - \omega_R)t - \Omega(t-\delta))} \right. \right.$$

$$\left. \left. \left. + \int_Z a_M(z)\big(F(z,t) + G(z,t)\big)dz \right) \right)^2 \right) dt$$

$$- \int_{3T_{CAM}/4}^{T_{CAM}} \left( \left( Re \left( A_R + A_D e^{i((\omega_O - \omega_R)t - \Omega(t-\delta))} \right. \right. \right.$$

$$\left. \left. + \int_Z a_M(z)\big(F(z,t) + G(z,t)\big)dz \right) \right)^2$$

$$+ \left( Im \left( A_R + A_D e^{i((\omega_O - \omega_R)t - \Omega(t-\delta))} \right. \right.$$

$$\left. \left. \left. + \int_Z a_M(z)\big(F(z,t) + G(z,t)\big)dz \right) \right)^2 \right) dt$$

Comme dans le mode de réalisation de la figure 5, on néglige les termes ne dépendant pas de $A_R$, l'énergie portée par le bras de référence dominant largement les autres termes. De plus, on néglige ici les termes dépendant de $A_D$. Du fait de leur composante haute fréquence indépendante de $\omega_{CAM}$, ces termes vont en effet être moyennés sur le temps d'intégration de chaque image, de l'ordre de $T_{CAM}/4$, et la valeur résiduelle annulée par la démodulation quatre phases (différence I0-I2 et I1-I3). On obtient alors :

[Math 66]

$$I = \int_0^{T_{CAM}/4} \left( 2Re(A_R)Re\left( \int_z a_M(z)\big(F(z,t) + G(z,t)\big)dz \right) \right.$$

$$\left. + 2Im(A_R)Im\left( \int_z a_M(z)\big(F(z,t) + G(z,t)\big)dz \right) \right) dt$$

$$- \int_{T_{CAM}/2}^{3T_{CAM}/4} \left( 2Re(A_R)Re\left( \int_z a_M(z)\big(F(z,t) + G(z,t)\big)dz \right) \right.$$

$$\left. + 2Im(A_R)Im\left( \int_z a_M(z)\big(F(z,t) + G(z,t)\big)dz \right) \right) dt$$

[Math 67]

$$Q = \int_{T_{CAM}/4}^{T_{CAM}/2} \left( 2Re(A_R)Re\left( \int_z a_M(z)\big(F(z,t) + G(z,t)\big)dz \right) \right.$$

$$\left. + 2Im(A_R)Im\left( \int_z a_M(z)\big(F(z,t) + G(z,t)\big)dz \right) \right) dt$$

$$- \int_{3T_{CAM}/4}^{T_{CAM}} \left( 2Re(A_R)Re\left( \int_z a_M(z)\big(F(z,t) + G(z,t)\big)dz \right) \right.$$

$$\left. + 2Im(A_R)Im\left( \int_z a_M(z)\big(F(z,t) + G(z,t)\big)dz \right) \right) dt$$

D'où :

[Math 68]

$$I = \int_0^{T_{CAM}/4} \left( 2Re(A_R) \int_z \left( Re\big(a_M(z)\big)Re\big(F(z,t) + G(z,t)\big) \right.\right.$$

$$\left. - Im\big(a_M(z)\big)Im\big(F(z,t) + G(z,t)\big) \right) dz$$

$$+ 2Im(A_R) \int_z \left( Re\big(a_M(z)\big)Im\big(F(z,t) + G(z,t)\big) \right.$$

$$\left.\left. + Im\big(a_M(z)\big)Re\big(F(z,t) + G(z,t)\big) \right) dz \right) dt$$

$$- \int_{T_{CAM}/2}^{3T_{CAM}/4} \left( 2Re(A_R) \int_z \left( Re\big(a_M(z)\big)Re\big(F(z,t) + G(z,t)\big) \right.\right.$$

$$\left. - Im\big(a_M(z)\big)Im\big(F(z,t) + G(z,t)\big) \right) dz$$

$$+ 2Im(A_R) \int_z \left( Re\big(a_M(z)\big)Im\big(F(z,t) + G(z,t)\big) \right.$$

$$\left.\left. + Im\big(a_M(z)\big)Re\big(F(z,t) + G(z,t)\big) \right) dz \right) dt$$

[Math 69]

$$Q = \int_{T_{CAM}/4}^{T_{CAM}/2} \left( 2Re(A_R) \int_z \left( Re\big(a_M(z)\big)Re\big(F(z,t) + G(z,t)\big) \right.\right.$$

$$\left. - Im\big(a_M(z)\big)Im\big(F(z,t) + G(z,t)\big) \right) dz$$

$$+ 2Im(A_R) \int_z \left( Re\big(a_M(z)\big)Im\big(F(z,t) + G(z,t)\big) \right.$$

$$\left.\left. + Im\big(a_M(z)\big)Re\big(F(z,t) + G(z,t)\big) \right) dz \right) dt$$

$$- \int_{3T_{CAM}/4}^{T_{CAM}} \left( 2Re(A_R) \int_z \left( Re\big(a_M(z)\big)Re\big(F(z,t) + G(z,t)\big) \right.\right.$$

$$\left. - Im\big(a_M(z)\big)Im\big(F(z,t) + G(z,t)\big) \right) dz$$

$$+ 2Im(A_R) \int_z \left( Re\big(a_M(z)\big)Im\big(F(z,t) + G(z,t)\big) \right.$$

$$\left.\left. + Im\big(a_M(z)\big)Re\big(F(z,t) + G(z,t)\big) \right) dz \right) dt$$

On réécrit alors les termes I et Q dans la forme décrite en relation avec la figure 5 :

[Math 70]

$$I = \int_z Re(a_M(z)) \left( \int_0^{T_{CAM}/4} \left( 2Re(A_R)Re(F(z,t) + G(z,t)) \right. \right.$$

$$\left. + 2Im(A_R)Im(F(z,t) + G(z,t)) \right) dt$$

$$- \int_{T_{CAM}/2}^{3T_{CAM}/4} \left( 2Re(A_R)Re(F(z,t) + G(z,t)) \right.$$

$$\left. \left. + 2Im(A_R)Im(F(z,t) + G(z,t)) \right) dt \right) dz$$

$$- \int_z Im(a_M(z)) \left( \int_0^{T_{CAM}/4} \left( 2Re(A_R)Im(F(z,t) + G(z,t)) \right. \right.$$

$$\left. - 2Im(A_R)Re(F(z,t) + G(z,t)) \right) dt$$

$$- \int_{T_{CAM}/2}^{3T_{CAM}/4} \left( 2Re(A_R)Im(F(z,t) + G(z,t)) \right.$$

$$\left. \left. - 2Im(A_R)Re(F(z,t) + G(z,t)) \right) dt \right) dz$$

[Math 71]

$$Q = \int_z Re(a_M(z)) \left( \int_{T_{CAM}/4}^{T_{CAM}/2} \left( 2Re(A_R)Re(F(z,t) + G(z,t)) \right. \right.$$

$$\left. + 2Im(A_R)Im(F(z,t) + G(z,t)) \right) dt$$

$$- \int_{3T_{CAM}/4}^{T_{CAM}} \left( 2Re(A_R)Re(F(z,t) + G(z,t)) \right.$$

$$\left. \left. + 2Im(A_R)Im(F(z,t) + G(z,t)) \right) dt \right) dz$$

$$- \int_z Im(a_M(z)) \left( \int_{T_{CAM}/4}^{T_{CAM}/2} \left( 2Re(A_R)Im(F(z,t) + G(z,t)) \right. \right.$$

$$\left. - 2Im(A_R)Re(F(z,t) + G(z,t)) \right) dt$$

$$- \int_{3T_{CAM}/4}^{T_{CAM}} \left( 2Re(A_R)Im(F(z,t) + G(z,t)) \right.$$

$$\left. \left. - 2Im(A_R)Re(F(z,t) + G(z,t)) \right) dt \right) dz$$

Pour chaque mesure de rang j, on réécrit ces équations comme suit :

[Math 72]

$$Q_j = \int_z Re\big(a_M(z)\big)C_j(z)\,dz - \int_z Im\big(a_M(z)\big)D_j(z)dz$$

[Math 73]

$$I_j = \int_z Re\big(a_M(z)\big)E_j(z)\,dz - \int_z Im\big(a_M(z)\big)F_j(z)dz$$

Avec :

[Math 74]

$$C_j(z) = \int_{T_{CAM}/4}^{T_{CAM}/2} \Big(2Re(A_R)Re\big(F_j(z,t) + G_j(z,t)\big)$$

$$+ 2Im(A_R)Im\big(F_j(z,t) + G_j(z,t)\big)\Big)dt$$

$$- \int_{3T_{CAM}/4}^{T_{CAM}} \Big(2Re(A_R)Re\big(F_j(z,t) + G_j(z,t)\big)$$

$$+ 2Im(A_R)Im\big(F_j(z,t) + G_j(z,t)\big)\Big)dt$$

[Math 75]

$$D_j(z) = \int_{T_{CAM}/4}^{T_{CAM}/2} \Big(2Re(A_R)Im\big(F_j(z,t) + G_j(z,t)\big)$$

$$- 2Im(A_R)Re\big(F_j(z,t) + G_j(z,t)\big)\Big)dt$$

$$- \int_{3T_{CAM}/4}^{T_{CAM}} \Big(2Re(A_R)Im\big(F_j(z,t) + G_j(z,t)\big)$$

$$- 2Im(A_R)Re\big(F_j(z,t) + G_j(z,t)\big)\Big)dt$$

[Math 76]

$$E_j(z) = \int_0^{T_{CAM}/4} \Bigg( 2Re(A_R)Re\Big(F_j(z,t) + G_j(z,t)\Big)$$

$$+ 2Im(A_R)Im\Big(F_j(z,t) + G_j(z,t)\Big)\Bigg) dt$$

$$- \int_{T_{CAM}/2}^{3T_{CAM}/4} \Bigg( 2Re(A_R)Re\Big(F_j(z,t) + G_j(z,t)\Big)$$

$$+ 2Im(A_R)Im\Big(F_j(z,t) + G_j(z,t)\Big)\Bigg) dt$$

[Math 77]

$$F_j(z) = \int_0^{T_{CAM}/4} \Bigg( 2Re(A_R)Im\Big(F_j(z,t) + G_j(z,t)\Big)$$

$$- 2Im(A_R)Re\Big(F_j(z,t) + G_j(z,t)\Big)\Bigg) dt$$

$$- \int_{T_{CAM}/2}^{3T_{CAM}/4} \Bigg( 2Re(A_R)Im\Big(F_j(z,t) + G_j(z,t)\Big)$$

$$- 2Im(A_R)Re\Big(F_j(z,t) + G_j(z,t)\Big)\Bigg) dt$$

Et :

[Math 78]

$$F_j(t,z) = e^{i\left(\omega_{CAM}t - \omega_{US}z/v_{US} + \Phi_j(t-z/v_{US}) - \Omega_j(t-\delta)\right)}$$

[Math 79]

$$G_j(t,z) = e^{i\left((\omega_{CAM} - 2\omega_{US})t + \omega_{US}z/v_{US} - \Phi_j(t-z/v_{US}) - \Omega_j(t-\delta)\right)}$$

Chacune des M mesures obtenues peut être réécrite sous la forme $Y_j = I_j + iQ_j$, avec :

[Math 80]

$$Q_j = \sum_{k=1}^{L} \Bigg( Re\big(a_M(k.\Delta z)\big) \int_{(k-1).\Delta z}^{k.\Delta z} C_j(z)dz - Im\big(a_M(k.\Delta z)\big) \int_{(k-1).\Delta z}^{k.\Delta z} D_j(z)dz \Bigg)$$

[Math 81]

$$I_j = \sum_{k=1}^{L} \left( Re\big(a_M(k.\Delta z)\big) \int_{(k-1).\Delta z}^{k.\Delta z} E_j(z)dz - Im\big(a_M(k.\Delta z)\big) \int_{(k-1).\Delta z}^{k.\Delta z} F_j(z)dz \right)$$

On retrouve la forme de I et Q définie dans l'exemple de la figure 5 (équations Math 25 et Math 26). On peut ainsi employer la même méthode de construction des matrices de passage que dans l'exemple précédent (équations Math 27 à Math 38), en remplaçant le terme G(t,z) par F(z,t)+G(z,t), puis la même méthode de reconstruction du profil d'absorption $a_M$ (équation Math 42).

Le système décrit en relation avec la figure 6 permet, pour chacune des M mesures de la phase d'acquisition du profil d'absorption, de réaliser la démodulation quatre phases à l'aide de 4 images acquises via une caméra standard (avec une période d'acquisition de $T_{CAM}$/4 pour chaque image). Cette technique permet en outre l'utilisation de fréquences de modulation acoustique $F_{US}$ plus élevées.

La figure 7 représente de façon schématique un exemple d'un système d'imagerie acousto-optique selon un troisième mode de réalisation.

Dans ce mode de réalisation, on prévoit d'étendre la méthode décrite en relation avec la figure 6 à des applications de mesure de profondeur dans une scène.

Dans le système de la figure 7, l'échantillon 105 est remplacé par un objet réfléchissant 705, correspondant à une scène dans laquelle on souhaite mesurer des informations de profondeur. Dans cet exemple, il n'est pas prévu de marquage localisé d'une partie du faisceau objet. Autrement dit, il n'est pas prévu de transducteur ultrasonore agencé pour appliquer une onde mécanique localisée à une partie de la scène. Sur la figure 7, par souci de simplification, les moyens de séparation du faisceau émis par la source en un faisceau objet et un faisceau de référence n'ont pas été représentés.

La séquence de modulation $\Phi$ est appliquée au faisceau objet en amont de la scène 705, via le modulateur acousto-optique MAO1. La séquence de démodulation $\Omega$ est quant à elle appliquée au faisceau de référence via le modulateur acousto-optique MAO2.

Les modulateurs MAO1 et MAO2 sont choisis de façon à obtenir une fréquence de battement $f_B$ (égale ici à la valeur absolue de la différence $f_{MAO1}$-$f_{MAO2}$), compatible avec l'utilisation d'un capteur basse fréquence, par exemple tels que la fréquence $F_C=K*f_B$ soit comprise entre 20 Hz et 20 kHz.

A titre d'exemple numérique illustratif et non limitatif, dans le système de la figure 7, le capteur 609 peut avoir une fréquence d'acquisition $F_C$ de 8 kHz, et les décalages fréquentiels $f_{MO1}$ et $f_{MAO2}$ introduits par les modulateurs MAO1 et MAO2 peuvent être respectivement de 80, 001 MHz, 79,999 MHz, pour obtenir une fréquence de battement $f_B = f_{MAO1} - f_{MAO2} = 2$ kHz $= F_C/4$ (en considérant le cas K=4) .

Dans le mode de réalisation de la figure 7, le module du vecteur $a_M$ mesuré correspond au profil d'absorption du faisceau source le long du trajet optique entre la sortie du modulateur MAO1 et le capteur 609, correspondant principalement à l'absorption des éléments réfléchissants de la scène 705 le long du chemin de propagation optique (de nature balistique dans ce cas).

Le signal s(t) mesuré au niveau d'un pixel du capteur 609 à l'issue de l'interférence entre le bras objet et le bras de référence est donné par l'équation suivante :

[Math 82]

$$s(t) = |E_r(t) + E_s(t)|^2$$

Avec :

[Math 83]

$$E_r(t) = A_R e^{i\left(\omega_R t + \Omega(t-\delta)\right)}$$

Et :

[Math 84]

$$E_s(t) = \int_z a_M(z)e^{i(\omega_O(t-z/v_L)+\Phi(t-z/v_L))}dz$$

Avec $v_L$ la vitesse de propagation de la lumière dans le milieu, et :

[Math 85]

$$\omega_R = 2\pi(f_L + f_{MAO2})$$

[Math 86]

$$\omega_O = 2\pi(f_L + f_{MAO1})$$

[Math 87]

$$\omega_R - \omega_O = \omega_{CAM}$$

[Math 88]

$$\omega_{CAM} = 2\pi F_{CAM}$$

On a alors :

[Math 89]

$$s(t) = \left(\left|e^{i(\omega_R t+\Omega(t-\delta))}\right| . \left|A_R + \int_z a_M e^{i(\omega_O(t-z/v_L)+\Phi(t-z/v_L)-\omega_R t-\Omega(t-\delta))}dz\right|\right)^2$$

[Math 90]

$$s(t) = \left|A_R + \int_z a_M e^{i(\omega_{CAM}t-\omega_O z/v_L+\Phi(t-z/v_L)-\Omega(t-\delta))}dz\right|^2$$

Au niveau du capteur 609, on acquiert successivement K=4 images avec une durée d'acquisition $T_{CAM}/4$ par image. Les voies I et Q peuvent, comme précédemment, être exprimées comme suit :

[Math 91]

$$I = \int_0^{T_{CAM}/4} s(t)dt - \int_{T_{CAM}/2}^{3T_{CAM}/4} s(t)dt$$

[Math 92]

$$Q = \int_{T_{CAM}/4}^{T_{CAM}/2} s(t)dt - \int_{3T_{CAM}/4}^{T_{CAM}} s(t)dt$$

On définit une fonction F telle que :

[Math 93]

$$F(z,t) = e^{i(\omega_{CAM}t - \omega_O z/v_L + \Phi(t - z/v_L) - \Omega(t - \delta))}$$

On a alors :

[Math 94]

$$I = \int_0^{T_{CAM}/4} \left( \left( Re\left( A_R + \int_z a_M F(z,t)dz \right) \right)^2 + \left( Im\left( A_R + \int_z a_M F(z,t)dz \right) \right)^2 \right) dt$$

$$- \int_{T_{CAM}/2}^{3T_{CAM}/4} \left( \left( Re\left( A_R + \int_z a_M F(z,t)dz \right) \right)^2 \right.$$

$$\left. + \left( Im\left( A_R + \int_z a_M F(z,t)dz \right) \right)^2 \right) dt$$

[Math 95]

$$Q = \int_{T_{CAM}/4}^{T_{CAM}/2} \left( \left( Re\left( A_R + \int_z a_M F(z,t)dz \right) \right)^2 + \left( Im\left( A_R + \int_z a_M F(z,t)dz \right) \right)^2 \right) dt$$

$$- \int_{3T_{CAM}/4}^{T_{CAM}} \left( \left( Re\left( A_R + \int_z a_M F(z,t)dz \right) \right)^2 \right.$$

$$\left. + \left( Im\left( A_R + \int_z a_M F(z,t)dz \right) \right)^2 \right) dt$$

[Math 96]

$$I = \int_0^{T_{CAM}/4} \left( 2Re(A_R)Re\left(\int_z a_M F(z,t)dz\right) + 2Im(A_R)Im\left(\int_z a_M F(z,t)dz\right) \right) dt$$

$$- \int_{T_{CAM}/2}^{3T_{CAM}/4} \left( 2Re(A_R)Re\left(\int_z a_M F(z,t)dz\right) \right.$$

$$\left. + 2Im(A_R)Im\left(\int_z a_M F(z,t)dz\right) \right) dt$$

[Math 97]

$$Q = \int_{T_{CAM}/4}^{T_{CAM}/2} \left( 2Re(A_R)Re\left(\int_z a_M F(z,t)dz\right) + 2Im(A_R)Im\left(\int_z a_M F(z,t)dz\right) \right) dt$$

$$- \int_{3T_{CAM}/4}^{T_{CAM}} \left( 2Re(A_R)Re\left(\int_z a_M F(z,t)dz\right) \right.$$

$$\left. + 2Im(A_R)Im\left(\int_z a_M F(z,t)dz\right) \right) dt$$

D'où :

[Math 98]

$$I = \int_0^{T_{CAM}/4} \left( 2Re(A_R) \int_Z \Big( Re\big(a_M(z)\big)Re\big(F(z,t)\big) - Im\big(a_M(z)\big)Im\big(F(z,t)\big) \Big) dz \right.$$

$$+ 2Im(A_R) \int_Z \Big( Re\big(a_M(z)\big)Im\big(F(z,t)\big)$$

$$+ Im\big(a_M(z)\big)Re\big(F(z,t)\big) \Big) dz \Bigg) dt$$

$$- \int_{T_{CAM}/2}^{3T_{CAM}/4} \left( 2Re(A_R) \int_Z \Big( Re\big(a_M(z)\big)Re\big(F(z,t)\big) \right.$$

$$- Im\big(a_M(z)\big)Im\big(F(z,t)\big) \Big) dz$$

$$+ 2Im(A_R) \int_Z \Big( Re\big(a_M(z)\big)Im\big(F(z,t)\big)$$

$$+ Im\big(a_M(z)\big)Re\big(F(z,t)\big) \Big) dz \Bigg) dt$$

[Math 99]

$$Q = \int_{T_{CAM}/4}^{T_{CAM}/2} \left( 2Re(A_R) \int_Z \Big( Re\big(a_M(z)\big)Re\big(F(z,t)\big) - Im\big(a_M(z)\big)Im\big(F(z,t)\big) \Big) dz \right.$$

$$+ 2Im(A_R) \int_Z \Big( Re\big(a_M(z)\big)Im\big(F(z,t)\big)$$

$$+ Im\big(a_M(z)\big)Re\big(F(z,t)\big) \Big) dz \Bigg) dt$$

$$- \int_{3T_{CAM}/4}^{T_{CAM}} \left( 2Re(A_R) \int_Z \Big( Re\big(a_M(z)\big)Re\big(F(z,t)\big) \right.$$

$$- Im\big(a_M(z)\big)Im\big(F(z,t)\big) \Big) dz$$

$$+ 2Im(A_R) \int_Z \Big( Re\big(a_M(z)\big)Im\big(F(z,t)\big)$$

$$+ Im\big(a_M(z)\big)Re\big(F(z,t)\big) \Big) dz \Bigg) dt$$

Pour chaque mesure de rang j, on réécrit ces équations comme suit :

[Math 100]

$$Q_j = \int_z Re\big(a_M(z)\big)C_j(z)\,dz - \int_z Im\big(a_M(z)\big)D_j(z)dz$$

[Math 101]

$$I_j = \int_z Re\big(a_M(z)\big)E_j(z)\,dz - \int_z Im\big(a_M(z)\big)F_j(z)dz$$

Avec :

[Math 102]

$$C_j(z) = \int_{T_{CAM}/4}^{T_{CAM}/2} \left(2Re(A_R)Re\left(F_j(z,t)\right) + 2Im(A_R)Im\left(F_j(z,t)\right)\right)dt$$
$$- \int_{3T_{CAM}/4}^{T_{CAM}} \left(2Re(A_R)Re\left(F_j(z,t)\right) + 2Im(A_R)Im\left(F_j(z,t)\right)\right)dt$$

[Math 103]

$$D_j(z) = \int_{T_{CAM}/4}^{T_{CAM}/2} \left(2Re(A_R)Im\left(F_j(z,t)\right) - 2Im(A_R)Re\left(F_j(z,t)\right)\right)dt$$
$$- \int_{3T_{CAM}/4}^{T_{CAM}} \left(2Re(A_R)Im\left(F_j(z,t)\right) - 2Im(A_R)Re\left(F_j(z,t)\right)\right)dt$$

[Math 104]

$$E_j(z) = \int_0^{T_{CAM}/4} \left(2Re(A_R)Re\left(F_j(z,t)\right) + 2Im(A_R)Im\left(F_j(z,t)\right)\right)dt$$
$$- \int_{T_{CAM}/2}^{3T_{CAM}/4} \left(2Re(A_R)Re\left(F_j(z,t)\right) + 2Im(A_R)Im\left(F_j(z,t)\right)\right)dt$$

[Math 105]

$$F_j(z) = \int_0^{T_{CAM}/4} \left(2Re(A_R)Im\left(F_j(z,t)\right) - 2Im(A_R)Re\left(F_j(z,t)\right)\right)dt$$
$$- \int_{T_{CAM}/2}^{3T_{CAM}/4} \left(2Re(A_R)Im\left(F_j(z,t)\right) - 2Im(A_R)Re\left(F_j(z,t)\right)\right)dt$$

[Math 106]

$$F_j(z,t) = e^{i\left(\omega_{CAM}t - \omega_O z/v_L + \Phi_j(t-z/v_L) - \Omega_j(t-\delta)\right)}$$

On peut réécrire également :

[Math 107]

$$C_j(z) = 2Re(A_R)\left(\int_{T_{CAM}/4}^{T_{CAM}/2} Re\left(F_j(z,t)\right)dt - \int_{3T_{CAM}/4}^{T_{CAM}} Re\left(F_j(z,t)\right)dt\right)$$

$$+ 2Im(A_R)\left(\int_{T_{CAM}/4}^{T_{CAM}/2} Im\left(F_j(z,t)\right)dt - \int_{3T_{CAM}/4}^{T_{CAM}} Im\left(F_j(z,t)\right)dt\right)$$

[Math 108]

$$D_j(z) = 2Re(A_R)\left(\int_{T_{CAM}/4}^{T_{CAM}/2} Im\left(F_j(z,t)\right)dt - \int_{3T_{CAM}/4}^{T_{CAM}} Im\left(F_j(z,t)\right)dt\right)$$

$$- 2Im(A_R)\left(\int_{T_{CAM}/4}^{T_{CAM}/2} Re\left(F_j(z,t)\right)dt - \int_{3T_{CAM}/4}^{T_{CAM}} Re\left(F_j(z,t)\right)dt\right)$$

[Math 109]

$$E_j(z) = 2Re(A_R)\left(\int_{0}^{T_{CAM}/4} Re\left(F_j(z,t)\right)dt - \int_{T_{CAM}/2}^{3T_{CAM}/4} Re\left(F_j(z,t)\right)dt\right)$$

$$+ 2Im(A_R)\left(\int_{0}^{T_{CAM}/4} Im\left(F_j(z,t)\right)dt - \int_{T_{CAM}/2}^{3T_{CAM}/4} Im\left(F_j(z,t)\right)dt\right)$$

[Math 110]

$$F_j(z) = 2Re(A_R)\left(\int_{0}^{T_{CAM}/4} Im\left(F_j(z,t)\right)dt - \int_{T_{CAM}/2}^{3T_{CAM}/4} Im\left(F_j(z,t)\right)dt\right)$$

$$- 2Im(A_R)\left(\int_{0}^{T_{CAM}/4} Re\left(F_j(z,t)\right)dt - \int_{T_{CAM}/2}^{3T_{CAM}/4} Re\left(F_j(z,t)\right)dt\right)$$

On note :

[Math 111]

$$H_{13\,Re\,j}(z) = \int_{0}^{T_{CAM}/4} Re\left(F_j(z,t)\right)dt - \int_{T_{CAM}/2}^{3T_{CAM}/4} Re\left(F_j(z,t)\right)dt$$

[Math 112]

$$H_{13\,Im\,j}(z) = \int_0^{T_{CAM}/4} Im\left(F_j(z,t)\right) dt - \int_{T_{CAM}/2}^{3T_{CAM}/4} Im\left(F_j(z,t)\right) dt$$

[Math 113]

$$H_{24\,Re\,j}(z) = \int_{T_{CAM}/4}^{T_{CAM}/2} Re\left(F_j(z,t)\right) dt - \int_{3T_{CAM}/4}^{T_{CAM}} Re\left(F_j(z,t)\right) dt$$

[Math 114]

$$H_{24\,Im\,j}(z) = \int_{T_{CAM}/4}^{T_{CAM}/2} Im\left(F_j(z,t)\right) dt - \int_{3T_{CAM}/4}^{T_{CAM}} Im\left(F_j(z,t)\right) dt$$

On peut alors réécrire :

[Math 115]

$$C_j(z) = 2Re(A_R)H_{24\,Re\,j}(z) + 2Im(A_R)H_{24\,Im\,j}(z)$$

[Math 116]

$$D_j(z) = 2Re(A_R)H_{24\,Im\,j}(z) - 2Im(A_R)H_{24\,Re\,j}(z)$$

[Math 117]

$$E_j(z) = 2Re(A_R)H_{13\,Re\,j}(z) + 2Im(A_R)H_{13\,Im\,j}(z)$$

[Math 118]

$$F_j(z) = 2Re(A_R)H_{13\,Im\,j}(z) - 2Im(A_R)H_{13\,Re\,j}(z)$$

Chacune des M mesures obtenues peut, comme précédemment, être réécrite sous la forme $Y_j = I_j + iQ_j$, avec :

[Math 119]

$$Q_j = \sum_{k=1}^{L}\left(Re\left(a_M(k.\Delta z)\right)\int_{(k-1).\Delta z}^{k.\Delta z} C_j(z)dz - Im\left(a_M(k.\Delta z)\right)\int_{(k-1).\Delta z}^{k.\Delta z} D_j(z)dz\right)$$

[Math 120]

$$I_j = \sum_{k=1}^{L}\left(Re\left(a_M(k.\Delta z)\right)\int_{(k-1).\Delta z}^{k.\Delta z} E_j(z)dz - Im\left(a_M(k.\Delta z)\right)\int_{(k-1).\Delta z}^{k.\Delta z} F_j(z)dz\right)$$

Dans cet exemple, le pas $\Delta z$ sera de préférence choisi très inférieur à la longueur d'onde $\lambda_L$ avec $\lambda_L = (3*10^8)*2\pi/\omega 0$.

En considérant un profil d'absorption de référence $a_{Mref}$ le long du chemin de propagation de la lumière, on a :

[Math 121]

$$Q_{j\,Ref} = \sum_{k=1}^{L} \left( Re\left(a_{M\,Ref}(k.\Delta z)\right) \int_{(k-1).\Delta z}^{k.\Delta z} C_j(z)dz \right.$$

$$\left. - Im\left(a_{M\,Ref}(k.\Delta z)\right) \int_{(k-1).\Delta z}^{k.\Delta z} D_j(z)dz \right)$$

[Math 122]

$$I_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$\left. - Im\left(a_{M\,Ref}(k.\Delta z)\right) \int_{(k-1).\Delta z}^{k.\Delta z} F_j(z)dz \right)$$

Soit :

[Math 123]

$$Y_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$\left. - Im\left(a_{M\,Ref}(k.\Delta z)\right) \int_{(k-1).\Delta z}^{k.\Delta z} F_j(z)dz \right)$$

$$+ i \sum_{k=1}^{L} \left( Re\left(a_{M\,Ref}(k.\Delta z)\right) \int_{(k-1).\Delta z}^{k.\Delta z} C_j(z)dz \right.$$

$$\left. - Im\left(a_{M\,Ref}(k.\Delta z)\right) \int_{(k-1).\Delta z}^{k.\Delta z} D_j(z)dz \right)$$

[Math 124]

$$Y_{j\,Ref} = \sum_{k=1}^{L} \left( Re\left(a_{M\,Ref}(k.\Delta z)\right) \int_{(k-1).\Delta z}^{k.\Delta z} E_j(z)dz \right.$$

$$- Im\left(a_{M\,Ref}(k.\Delta z)\right) \int_{(k-1).\Delta z}^{k.\Delta z} F_j(z)dz$$

$$+ i\left( Re\left(a_{M\,Ref}(k.\Delta z)\right) \int_{(k-1).\Delta z}^{k.\Delta z} C_j(z)dz \right.$$

$$\left. \left. - Im\left(a_{M\,Ref}(k.\Delta z)\right) \int_{(k-1).\Delta z}^{k.\Delta z} D_j(z)dz \right) \right)$$

[Math 125]

$$Y_{j\,Ref} = \sum_{k=1}^{L} \left( Re\left(a_{M\,Ref}(k.\Delta z)\right) \int_{(k-1).\Delta z}^{k.\Delta z} \left(E_j(z) + iC_j(z)\right)dz \right.$$

$$\left. - Im\left(a_{M\,Ref}(k.\Delta z)\right) \int_{(k-1).\Delta z}^{k.\Delta z} \left(F_j(z) + iD_j(z)\right)dz \right)$$

[Math 126]

$$Y_{j\,Ref} = \sum_{k=1}^{L} \left( Re\left(a_{M\,Ref}(k.\Delta z)\right) \int_{(k-1).\Delta z}^{k.\Delta z} \left(2Re(A_R)H_{13\,Re\,j}(z) + 2Im(A_R)H_{13\,Im\,j}(z) \right.\right.$$

$$\left. + i\left(2Re(A_R)H_{24\,Re\,j}(z) + 2Im(A_R)H_{24\,Im\,j}(z)\right)\right)dz$$

$$- Im\left(a_{M\,Ref}(k.\Delta z)\right) \int_{(k-1).\Delta z}^{k.\Delta z} \left(2Re(A_R)H_{13\,Im\,j}(z) \right.$$

$$- 2Im(A_R)H_{13\,Re\,j}(z)$$

$$\left. \left. + i\left(2Re(A_R)H_{24\,Im\,j}(z) - 2Im(A_R)H_{24\,Re\,j}(z)\right)\right)dz \right)$$

[Math 127]

$$Y_{j\,Ref} = \sum_{k=1}^{L} \left( 2Re\left(a_{M\,Ref}(k.\Delta z)\right) Re(A_R) \int_{(k-1).\Delta z}^{k.\Delta z} \left(H_{13\,Re\,j}(z) + iH_{24\,Re\,j}(z)\right) dz \right.$$

$$+ 2Re\left(a_{M\,Ref}(k.\Delta z)\right) Im(A_R) \int_{(k-1).\Delta z}^{k.\Delta z} \left(H_{13\,Im\,j}(z) + iH_{24\,Im\,j}(z)\right) dz$$

$$- 2Im\left(a_{M\,Ref}(k.\Delta z)\right) Re(A_R) \int_{(k-1).\Delta z}^{k.\Delta z} \left(H_{13\,Im\,j}(z) + iH_{24\,Im\,j}(z)\right) dz$$

$$\left. + 2Im\left(a_{M\,Ref}(k.\Delta z)\right) Im(A_R) \int_{(k-1).\Delta z}^{k.\Delta z} \left(H_{13\,Re\,j}(z) + iH_{24\,Re\,j}(z)\right) dz \right)$$

[Math 128]

$$Y_{j\,Ref} = 2\sum_{k=1}^{L} \left( Re\left(a_{M\,Ref}(k.\Delta z)\right) \left( Re(A_R) \int_{(k-1).\Delta z}^{k.\Delta z} \left(H_{13\,Re\,j}(z) + iH_{24\,Re\,j}(z)\right) dz \right. \right.$$

$$\left. + Im(A_R) \int_{(k-1).\Delta z}^{k.\Delta z} \left(H_{13\,Im\,j}(z) + iH_{24\,Im\,j}(z)\right) dz \right)$$

$$- Im\left(a_{M\,Ref}(k.\Delta z)\right) \left( Re(A_R) \int_{(k-1).\Delta z}^{k.\Delta z} \left(H_{13\,Im\,j}(z) + iH_{24\,Im\,j}(z)\right) dz \right.$$

$$\left. \left. - Im(A_R) \int_{(k-1).\Delta z}^{k.\Delta z} \left(H_{13\,Re\,j}(z) + iH_{24\,Re\,j}(z)\right) dz \right) \right)$$

En ayant identifié la relation entre les mesures et le profil d'absorption selon l'équation Math 128, on peut établir la relation matricielle suivante :

[Math 129]

$$Y_j = \mathcal{A}_{Re\,j,k}Re(a_M) - \mathcal{A}_{Im\,j,k}Im(a_M)$$

Où $A_{Re}$ et $A_{Im}$ sont deux matrices ayant chacune M rangées et L colonnes, et $a_M$ est un vecteur de L rangées et une colonne.
Avec :

[Math 130]

$$\mathcal{A}_{Re\,j,k} = 2\left(Re(A_R)\int_{(k-1).\Delta z}^{k.\Delta z}\left(H_{13\,Re\,j}(z) + iH_{24\,Re\,j}(z)\right)dz\right.$$

$$\left. + Im(A_R)\int_{(k-1).\Delta z}^{k.\Delta z}\left(H_{13\,Im\,j}(z) + iH_{24\,Im\,j}(z)\right)dz\right)$$

[Math 131]

$$\mathcal{A}_{Im\,j,k} = 2\left(Re(A_R)\int_{(k-1).\Delta z}^{k.\Delta z}\left(H_{13\,Im\,j}(z) + iH_{24\,Im\,j}(z)\right)dz\right.$$

$$\left. - Im(A_R)\int_{(k-1).\Delta z}^{k.\Delta z}\left(H_{13\,Re\,j}(z) + iH_{24\,Re\,j}(z)\right)dz\right)$$

On peut alors employer la même méthode de reconstruction du profil d'absorption $a_M$ que dans les exemples précédents (équation Math 42).

On notera que le mode de réalisation de la figure 5 peut également être adapté pour des applications de mesure de profondeur dans une scène. Dans ce cas, le montage sera similaire à celui de la figure 7, mais avec un seul modulateur acoustique MAO1, placé sur le bras objet, permettant d'appliquer la séquence de modulation $\Phi$, et en remplaçant le capteur basse fréquence 609 de la figure 6 par un capteur synchrone 109 du type décrit en relation avec la figure 5, permettant d'appliquer la séquence de démodulation $\Omega$ dans le domaine électronique.

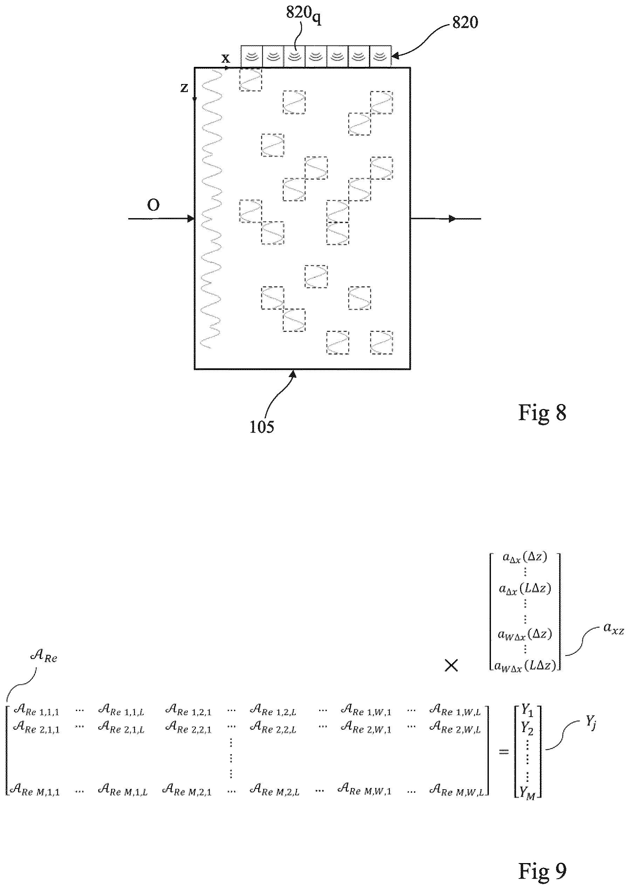

La figure 8 illustre le fonctionnement d'une variante de réalisation du système d'imagerie acousto-optique de la figure 5.

Dans l'exemple de la figure 5, on a proposé un système d'acquisition d'un profil d'absorption $a_M$ à une dimension. Pour cela, un unique transducteur ultrasonore (ou un unique groupe de transducteurs ultrasonores commandés simultanément) 120 (figure 5) applique une onde sinusoïdale de marquage se propageant de façon balistique dans une zone localisée de l'échantillon, par exemple une zone en forme de colonne. La direction de propagation de l'onde acoustique de marquage correspond à la direction de reconstruction du profil d'absorption $a_M$ (la direction z dans les formules détaillées ci-dessus).

Dans la variante de la figure 8, on prévoit d'étendre le principe de mesure détaillé en relation avec la figure 5 à la reconstruction d'un profil d'absorption à deux dimensions, x et z, par exemple orthogonales.

Pour cela, on utilise comme précédemment une onde acoustique d'excitation sinusoïdale de référence présentant des sauts de phase définis selon un motif aléatoire ou pseudo-aléatoire $\Phi$. Cette onde est émise non plus à l'aide d'un seul transducteur selon une trajectoire unique, mais au moyen d'une barrette 820 de W transducteurs élémentaires $820_q$ répartis spatialement selon l'axe x, avec W entier supérieur ou égal à 2 et q entier allant de 1 à W. Dans cet exemple, les W transducteurs $820_q$ sont alignés selon l'axe x, et chaque transducteur $820_q$ est agencé pour émettre selon une direction principale de propagation parallèle à l'axe z.

Dans la variante de la figure 8, l'onde acoustique d'excitation est émise par fractions par les différents transducteurs de manière aléatoire ou pseudo-aléatoire, selon une fonction $\gamma$. Chaque fraction d'onde émise par un transducteur correspond de préférence à une période $T_{US}$ unique ou à un nombre entier de périodes $T_{US}$ de l'onde acoustique d'excitation. Une même fraction de l'onde acoustique d'excitation peut être émise de façon simultanée par plusieurs transducteurs $820_q$ (c'est-à-dire simultanément depuis plusieurs positions x).

On peut alors décrire le principe de mesure à l'aide des équations suivantes, en utilisant le formalisme employé précédemment. On désigne ci-après par $\Delta x$ le pas inter-transducteurs, et par $\gamma_x$ une séquence binaire aléatoire ou pseudo-aléatoire permettant de modéliser l'activation du transducteur situé à la position x en fonction du temps. Le terme $a_x(z)$ est un vecteur colonne de L valeurs représentant le profil d'absorption selon z à la position x.

L'onde du faisceau de référence et l'onde du faisceau objet s'écrivent :

[Math 132]

$$E_r(t) = A_R e^{i\omega_L t}$$

[Math 133]

$$E_{s\,j}(t) = A_D e^{i\omega_L t}$$

$$+ \sum_{q=1}^{W} \left( \int_z a_{q\Delta x}(z) \Upsilon_{q\Delta x,j}(t - z/v_{US}) e^{i\left(\omega_L t + \omega_{US}(t - z/v_{US}) + \pi\Phi_j(t - z/v_{US})\right)} dz \right.$$

$$+ \left. \int_z a_{q\Delta x}(z) \Upsilon_{q\Delta x,j}(t - z/v_{US}) e^{i\left(\omega_L t - \omega_{US}(t - z/v_{US}) - \pi\Phi_j(t - z/v_{US})\right)} dz \right)$$

L'interférence $s_j(t)$ mesurée au niveau du capteur correspondant à une mesure de rang j s'écrit alors :

[Math 134]

$$s_j(t) = \left| E_r(t) + E_{s\,j}(t) \right|^2$$

[Math 135]

$$s_j(t) = \left| A_R e^{i\omega_L t} + A_D e^{i\omega_L t} \right.$$

$$+ \sum_{q=1}^{W} \left( \int_z a_{q\Delta x}(z) \Upsilon_{q\Delta x,j}(t - z/v_{US}) e^{i\left(\omega_L t + \omega_{US}(t - z/v_{US}) + \pi\Phi_j(t - z/v_{US})\right)} dz \right.$$

$$+ \left. \left. \int_z a_{q\Delta x}(z) \Upsilon_{q\Delta x,j}(t - z/v_{US}) e^{i\left(\omega_L t - \omega_{US}(t - z/v_{US}) - \pi\Phi_j(t - z/v_{US})\right)} dz \right) \right|^2$$

[Math 136]

$$s_j(t) = \left( \left| e^{i\omega_L t} \right| \Big| A_R + A_D \right.$$

$$+ \sum_{q=1}^{W} \left( \int_z a_{q\Delta x}(z) \Upsilon_{q\Delta x,j}(t - z/v_{US}) e^{i\left( \omega_{US}(t-z/v_{US}) + \pi\Phi_j(t-z/v_{US}) \right)} dz \right.$$

$$\left. \left. + \int_z a_{q\Delta x}(z) \Upsilon_{q\Delta x,j}(t - z/v_{US}) e^{i\left( -\omega_{US}(t-z/v_{US}) - \pi\Phi_j(t-z/v_{US}) \right)} dz \right) \right| \right)^2$$

[Math 137]

$$s_j(t) = \Big| A_R + A_D$$

$$+ 2 \sum_{q=1}^{W} \left( \int_z a_{q\Delta x}(z) \Upsilon_{q\Delta x,j}(t - z/v_{US}) \cos \Big( \omega_{US}(t - z/v_{US}) \right.$$

$$\left. + \pi\Phi_j(t - z/v_{US}) \Big) dz \right) \Big|^2$$

On mesure au niveau du capteur, par démodulation quatre phases :

[Math 138]

$$I_j = \sum_{p=1}^{N} \Pi_j(p) \left( \int_{pT_{US}}^{pT_{US}+T_{US}/4} s_j(t)dt - \int_{pT_{US}+T_{US}/2}^{pT_{US}+3T_{US}/4} s_j(t)dt \right)$$

[Math 139]

$$Q_j = \sum_{p=1}^{N} \Pi_j(p) \left( \int_{pT_{US}+T_{US}/4}^{pT_{US}+T_{US}/2} s_j(t)dt - \int_{pT_{US}+3T_{US}/4}^{pT_{US}+T_{US}} s_j(t)dt \right)$$

Avec :

[Math 140]

$$\Pi_j(p) = 2 \left( \Omega_j(p) - \frac{1}{2} \right)$$

D'où par exemple pour la voie Q :

[Math 141]

$$
\begin{aligned}
Q_j = \sum_{p=1}^{N} \Pi_j(p) \Bigg( & \int_{pT_{US}+T_{US}/4}^{pT_{US}+T_{US}/2} \Bigg( Re\Bigg( A_D + A_R \\
& + 2\sum_{q=1}^{W}\Bigg(\int_z a_{q\Delta x}(z)\Upsilon_{q\Delta x,j}(t-z/v_{US})\cos\Big(\omega_{US}(t-z/v_{US})+\pi\Phi_j(t \\
& - z/v_{US})\Big)dz\Bigg)\Bigg)\Bigg)^2 dt \\
& - \int_{pT_{US}+3T_{US}/4}^{pT_{US}+T_{US}} \Bigg( Re\Bigg( A_D + A_R \\
& + 2\sum_{q=1}^{W}\Bigg(\int_z a_{q\Delta x}(z)\Upsilon_{q\Delta x,j}(t-z/v_{US})\cos\Big(\omega_{US}(t-z/v_{US})+\pi\Phi_j(t \\
& - z/v_{US})\Big)dz\Bigg)\Bigg)\Bigg)^2 dt \\
& + \int_{pT_{US}+T_{US}/4}^{pT_{US}+T_{US}/2} \Bigg( Im\Bigg( A_D + A_R \\
& + 2\sum_{q=1}^{W}\Bigg(\int_z a_{q\Delta x}(z)\Upsilon_{q\Delta x,j}(t-z/v_{US})\cos\Big(\omega_{US}(t-z/v_{US})+\pi\Phi_j(t \\
& - z/v_{US})\Big)dz\Bigg)\Bigg)\Bigg)^2 dt \\
& - \int_{pT_{US}+3T_{US}/4}^{pT_{US}+T_{US}} \Bigg( Im\Bigg( A_D + A_R \\
& + 2\sum_{q=1}^{W}\Bigg(\int_z a_{q\Delta x}(z)\Upsilon_{q\Delta x,j}(t-z/v_{US})\cos\Big(\omega_{US}(t-z/v_{US})+\pi\Phi_j(t \\
& - z/v_{US})\Big)dz\Bigg)\Bigg)\Bigg)^2 dt \Bigg)
\end{aligned}
$$

On néglige les termes ne comprenant pas la composante $A_R$ du bras de référence, et on définit le terme suivant :

[Math 142]

$$G_j(t,z) = cos\left(\omega_{US}(t - z/v_{US}) + \pi\Phi_j(t - z/v_{US})\right)$$

L'équation Math 141 se simplifie alors comme suit :

[Math 143]

$$Q_j = \sum_{p=1}^{N} \Pi_j(p) \left( \int_{pT_{US}+T_{US}/4}^{pT_{US}+T_{US}/2} 4Re(A_R) \left( \sum_{q=1}^{W} \left( \int_z Re\left(a_{q\Delta x}(z)Y_{q\Delta x,j}(t \right. \right. \right.$$

$$\left. \left. \left. - z/v_{US})G_j(t,z)\right) dz \right) \right) dt$$

$$- \int_{pT_{US}+3T_{US}/4}^{pT_{US}+T_{US}} 4Re(A_R) \left( \sum_{q=1}^{W} \left( \int_z Re\left(a_{q\Delta x}(z)Y_{q\Delta x,j}(t \right. \right. \right.$$

$$\left. \left. \left. - z/v_{US})G_j(t,z)\right) dz \right) \right) dt$$

$$+ \int_{pT_{US}+T_{US}/4}^{pT_{US}+T_{US}/2} 4Im(A_R) \left( \sum_{q=1}^{W} \left( \int_z Im\left(a_{q\Delta x}(z)Y_{q\Delta x,j}(t \right. \right. \right.$$

$$\left. \left. \left. - z/v_{US})G_j(t,z)\right) dz \right) \right) dt$$

$$- \int_{pT_{US}+3T_{US}/4}^{pT_{US}+T_{US}} 4Im(A_R) \left( \sum_{q=1}^{W} \left( \int_z Im\left(a_{q\Delta x}(z)Y_{q\Delta x,j}(t \right. \right. \right.$$

$$\left. \left. \left. - z/v_{US})G_j(t,z)\right) dz \right) \right) dt \right)$$

On a alors :

[Math 144]

$$Q_j = \sum_{p=1}^{N} \Pi_j(p) \left( \int_{pT_{US}+T_{US}/4}^{pT_{US}+T_{US}/2} 4Re(A_R) \left( \sum_{q=1}^{W} \left( \int_z Re\left(a_{q\Delta x}(z)\Upsilon_{q\Delta x,j}(t\right.\right.\right.$$

$$\left.\left.\left. - z/v_{US})\right) Re\left(G_j(t,z)\right) dz \right) \right) dt$$

$$- \int_{pT_{US}+3T_{US}/4}^{pT_{US}+T_{US}} 4Re(A_R) \left( \sum_{q=1}^{W} \left( \int_z Re\left(a_{q\Delta x}(z)\Upsilon_{q\Delta x,j}(t\right.\right.\right.$$

$$\left.\left.\left. - z/v_{US})\right) Re\left(G_j(t,z)\right) dz \right) \right) dt$$

$$+ \int_{pT_{US}+T_{US}/4}^{pT_{US}+T_{US}/2} 4Im(A_R) \left( \sum_{q=1}^{W} \left( \int_z Im\left(a_{q\Delta x}(z)\Upsilon_{q\Delta x,j}(t\right.\right.\right.$$

$$\left.\left.\left. - z/v_{US})\right) Re\left(G_j(t,z)\right) dz \right) \right) dt$$

$$- \int_{pT_{US}+3T_{US}/4}^{pT_{US}+T_{US}} 4Im(A_R) \left( \sum_{q=1}^{W} \left( \int_z Im\left(a_{q\Delta x}(z)\Upsilon_{q\Delta x,j}(t\right.\right.\right.$$

$$\left.\left.\left. - z/v_{US})\right) Re\left(G_j(t,z)\right) \right) \right) dt \right)$$

[Math 145]

$$Q_j = \sum_{q=1}^{W} \left( \int_z Re\left(a_{q\Delta x}(z)\right) \left( 4Re(A_R) \left( \sum_{p=1}^{N} \Pi_j(p) \left( \int_{pT_{US}+T_{US}/4}^{pT_{US}+T_{US}/2} \Upsilon_{q\Delta x,j}(t \right. \right. \right. \right.$$

$$\left. - z/v_{US})Re\left(G_j(t,z)\right) dt \right.$$

$$\left. - \int_{pT_{US}+3T_{US}/4}^{pT_{US}+T_{US}} \Upsilon_{q\Delta x,j}(t - z/v_{US})Re\left(G_j(t,z)\right) dt \right) \right) \right) dz$$

$$+ \int_z Im\left(a_{q\Delta x}(z)\right) \left( 4Im(A_R) \left( \sum_{p=1}^{N} \Pi_j(p) \left( \int_{pT_{US}+T_{US}/4}^{pT_{US}+T_{US}/2} \Upsilon_{q\Delta x,j}(t \right. \right. \right.$$

$$\left. - z/v_{US})Re\left(G_j(t,z)\right) dt \right.$$

$$\left. \left. \left. - \int_{pT_{US}+3T_{US}/4}^{pT_{US}+T_{US}} \Upsilon_{q\Delta x,j}(t - z/v_{US})Re\left(G_j(t,z)\right) dt \right) \right) \right) dz \right)$$

De façon similaire, on peut montrer que :

[Math 146]

$$I_j = \sum_{q=1}^{W} \left( \int_z Re\left(a_{q\Delta x}(z)\right) \left( 4Re(A_R) \left( \sum_{p=1}^{N} \Pi_j(p) \left( \int_{pT_{US}}^{pT_{US}+T_{US}/4} \Upsilon_{q\Delta x,j}(t \right. \right. \right. \right.$$

$$- z/v_{US})Re\left(G_j(t,z)\right) dt$$

$$\left. \left. \left. \left. - \int_{pT_{US}+T_{US}/2}^{pT_{US}+3T_{US}/4} \Upsilon_{q\Delta x,j}(t - z/v_{US})Re\left(G_j(t,z)\right) dt \right) \right) \right) dz \right.$$

$$+ \int_z Im\left(a_{q\Delta x}(z)\right) \left( 4Im(A_R) \left( \sum_{p=1}^{N} \Pi_j(p) \left( \int_{pT_{US}}^{pT_{US}+T_{US}/4} \Upsilon_{q\Delta x,j}(t \right. \right. \right.$$

$$- z/v_{US})Re\left(G_j(t,z)\right) dt$$

$$\left. \left. \left. \left. \left. - \int_{pT_{US}+T_{US}/2}^{pT_{US}+3T_{US}/4} \Upsilon_{q\Delta x,j}(t - z/v_{US})Re\left(G_j(t,z)\right) dt \right) \right) \right) dz \right)$$

Pour chacune des M mesures de la phase d'acquisition du profil d'absorption a$_M$, on peut écrire :

[Math 147]

$$Q_j = \sum_{q=1}^{W} \left( \int_z Re\left(a_{q\Delta x}(z)\right) C_{jq}(z)\, dz + \int_z Im\left(a_{q\Delta x}(z)\right) D_{jq}(z)dz \right)$$

[Math 148]

$$I_j = \sum_{q=1}^{W} \left( \int_z Re\left(a_{q\Delta x}(z)\right) E_{jq}(z)\, dz + \int_z Im\left(a_{q\Delta x}(z)\right) F_{jq}(z)dz \right)$$

Avec :

[Math 149]

$$C_{jq}(z) = 4Re(A_R) \sum_{p=1}^{N} \Pi_j(p) \left( \int_{pT_{US}+T_{US}/4}^{pT_{US}+T_{US}/2} \Upsilon_{q\Delta x,j}(t - z/v_{US}) \cos\left(\omega_{US}(t - z/v_{US})\right.\right.$$

$$\left. + \pi\Phi_j(t - z/v_{US})\right) dt$$

$$- \int_{pT_{US}+3T_{US}/4}^{pT_{US}+T_{US}} \Upsilon_{q\Delta x,j}(t - z/v_{US}) \cos\left(\omega_{US}(t - z/v_{US})\right.$$

$$\left.\left. + \pi\Phi_j(t - z/v_{US})\right) dt\right)$$

[Math 150]

$$D_{jq}(z) = 4Im(A_R) \left( \sum_{p=1}^{N} \Pi_j(p) \left( \int_{pT_{US}+T_{US}/4}^{pT_{US}+T_{US}/2} \Upsilon_{q\Delta x,j}(t - z/v_{US}) \cos\left(\omega_{US}(t - z/v_{US})\right.\right.\right.$$

$$\left. + \pi\Phi_j(t - z/v_{US})\right) dt$$

$$- \int_{pT_{US}+3T_{US}/4}^{pT_{US}+T_{US}} \Upsilon_{q\Delta x,j}(t - z/v_{US}) \cos\left(\omega_{US}(t - z/v_{US})\right.$$

$$\left.\left.\left. + \pi\Phi_j(t - z/v_{US})\right) dt\right)\right)$$

[Math 151]

$$E_{jq}(z) = 4Re(A_R) \left( \sum_{p=1}^{N} \Pi_j(p) \left( \int_{pT_{US}}^{pT_{US}+T_{US}/4} \Upsilon_{q\Delta x,j}(t - z/v_{US}) \cos\left(\omega_{US}(t - z/v_{US})\right.\right.\right.$$

$$\left. + \pi\Phi_j(t - z/v_{US})\right) dt$$

$$- \int_{pT_{US}+T_{US}/2}^{pT_{US}+3T_{US}/4} \Upsilon_{q\Delta x,j}(t - z/v_{US}) \cos\left(\omega_{US}(t - z/v_{US})\right.$$

$$\left.\left.\left. + \pi\Phi_j(t - z/v_{US})\right) dt\right)\right)$$

[Math 152]

$$F_{jq}(z) = 4Im(A_R)\left(\sum_{p=1}^{N}\Pi_j(p)\left(\int_{pT_{US}}^{pT_{US}+T_{US}/4}\Upsilon_{q\Delta x,j}(t-z/v_{US})cos\left(\omega_{US}(t-z/v_{US})\right.\right.\right.$$

$$\left.+\pi\Phi_j(t-z/v_{US})\right)dt$$

$$-\int_{pT_{US}+T_{US}/2}^{pT_{US}+3T_{US}/4}\Upsilon_{q\Delta x,j}(t-z/v_{US})cos\left(\omega_{US}(t-z/v_{US})\right.$$

$$\left.\left.\left.+\pi\Phi_j(t-z/v_{US})\right)dt\right)\right)$$

Chaque mesure peut être réécrite sous la forme $Y_j=I_j + iQ_j$, avec :

[Math 153]

$$Q_j = \sum_{q=1}^{W}\sum_{k=1}^{L}\left(Re\left(a_{q\Delta x}(k\Delta z)\right)\int_{(k-1)\Delta z}^{k\Delta z}C_{jq}(z)dz + Im\left(a_{q\Delta x}(k\Delta z)\right)\int_{(k-1)\Delta z}^{k\Delta z}D_{jq}(z)dz\right)$$

[Math 154]

$$I_j = \sum_{q=1}^{W}\sum_{k=1}^{L}\left(Re\left(a_{q\Delta x}(k\Delta z)\right)\int_{(k-1)\Delta z}^{k\Delta z}E_{jq}(z)dz + Im\left(a_{q\Delta x}(k\Delta z)\right)\int_{(k-1)\Delta z}^{k\Delta z}F_{jq}(z)dz\right)$$

Le terme $\Delta z$ représente ici une fraction de la longueur d'onde $\lambda_{US}=T_{US}*\upsilon_{US}$ du signal ultrasonore d'excitation de pulsation $\omega_{US}$, choisie de façon à pouvoir considérer l'absorption constante sur un intervalle $\Delta z$. Selon les notations utilisées, le milieu considéré est d'épaisseur $L*\Delta z$.

Afin de construire la matrice de passage du système, on réécrit les termes I et Q comme suit, en considérant une contribution de référence $a_{refx}(Z)$ de chaque position x de la zone de marquage acoustique, permettant par exemple de modéliser l'efficacité de marquage acoustique à chaque position de la zone de marquage :

[Math 155]

$$Q_{j\,Ref} = \sum_{q=1}^{W}\sum_{k=1}^{L}\left(Re\left(a_{Ref\,q\Delta x}(k\Delta z)\right)\int_{(k-1)\Delta z}^{k\Delta z}C_{jq}(z)dz\right.$$

$$\left.+ Im\left(a_{Ref\,q\Delta x}(k\Delta z)\right)\int_{(k-1)\Delta z}^{k\Delta z}D_{jq}(z)dz\right)$$

[Math 156]

$$I_{j\,Ref} = \sum_{q=1}^{W} \sum_{k=1}^{L} \left( Re\left(a_{Ref\,q\Delta x}(k\Delta z)\right) \int_{(k-1)\Delta z}^{k\Delta z} E_{jq}(z)dz \right.$$

$$\left. + Im\left(a_{Ref\,q\Delta x}(k\Delta z)\right) \int_{(k-1)\Delta z}^{k\Delta z} F_{jq}(z)dz \right)$$

D'où :

[Math 157]

$$Y_{j\,Ref} = \sum_{q=1}^{W} \sum_{k=1}^{L} \left( \left( Re\left(a_{Ref\,q\Delta x}(k\Delta z)\right) \int_{(k-1)\Delta z}^{k\Delta z} E_{jq}(z)dz \right. \right.$$

$$\left. + Im\left(a_{Ref\,q\Delta x}(k\Delta z)\right) \int_{(k-1)\Delta z}^{k\Delta z} F_{jq}(z)dz \right)$$

$$+ i\left( Re\left(a_{Ref\,q\Delta x}(k\Delta z)\right) \int_{(k-1)\Delta z}^{k\Delta z} C_{jq}(z)dz \right.$$

$$\left. \left. + Im\left(a_{Ref\,q\Delta x}(k\Delta z)\right) \int_{(k-1)\Delta z}^{k\Delta z} D_{jq}(z)dz \right) \right)$$

[Math 158]

$$Y_{j\,Ref} = \sum_{q=1}^{W} \sum_{k=1}^{L} \left( 4Re(A_R)Re\left(a_{Ref\,q\Delta x}(k\Delta z)\right) \int_{(k-1)\Delta z}^{k\Delta z} \left(S13_{jq}(z) + iS24_{jq}(z)\right)dz \right.$$

$$\left. + 4Im(A_R)Im\left(a_{Ref\,q\Delta x}(k\Delta z)\right) \int_{(k-1)\Delta z}^{k\Delta z} \left(S13_{jq}(z) + iS24_{jq}(z)\right)dz \right)$$

Avec :

[Math 159]

$$S13_{jq}(z) = \sum_{p=1}^{N} \Pi_j(p) \left( \int_{pT_{US}}^{pT_{US}+T_{US}/4} \Upsilon_{q\Delta x,j}(t - z/v_{US}) cos \left( \omega_{US}(t - z/v_{US}) \right.\right.$$

$$\left. + \pi \Phi_j(t - z/v_{US}) \right) dt$$

$$- \int_{pT_{US}+T_{US}/2}^{pT_{US}+3T_{US}/4} \Upsilon_{q\Delta x,j}(t - z/v_{US}) cos \left( \omega_{US}(t - z/v_{US}) \right.$$

$$\left.\left. + \pi \Phi_j(t - z/v_{US}) \right) dt \right)$$

[Math 160]

$$S24_{jq}(z) = \sum_{p=1}^{N} \Pi_j(p) \left( \int_{pT_{US}+T_{US}/4}^{pT_{US}+T_{US}/2} \Upsilon_{q\Delta x,j}(t - z/v_{US}) cos \left( \omega_{US}(t - z/v_{US}) \right.\right.$$

$$\left. + \pi \Phi_j(t - z/v_{US}) \right) dt$$

$$- \int_{pT_{US}+3T_{US}/4}^{pT_{US}+T_{US}} \Upsilon_{q\Delta x,j}(t - z/v_{US}) cos \left( \omega_{US}(t - z/v_{US}) \right.$$

$$\left.\left. + \pi \Phi_j(t - z/v_{US}) \right) dt \right)$$

Soit :

[Math 161]

$$Y_{j\,Ref} = \sum_{q=1}^{W} \sum_{k=1}^{L} \left( \left( 4Re(A_R)Re \left( a_{Ref\,q\Delta x}(k\Delta z) \right) \right.\right.$$

$$\left.\left. + 4Im(A_R)Im \left( a_{Ref\,q\Delta x}(k\Delta z) \right) \right) \int_{(k-1)\Delta z}^{k\Delta z} \left( S13_{jq}(z) + iS24_{jq}(z) \right) dz \right)$$

En ayant identifié la relation entre les mesures et le profil d'absorption selon l'équation Math 161, on peut établir la relation matricielle suivante :

[Math 162]

$$Y_j = \mathcal{A}_{Re}Re(a_{xz}) + \mathcal{A}_{Im}Im(a_{xz})$$

Où $a_{xz}$ est un vecteur de L*W rangées et une colonne correspondant à la concaténation des W profils d'absorption de L points chacun, correspondant respectivement aux W positions de mesure sur l'axe x, tel que :

[Math 163]

$$a_{xz} = \left(a_{\Delta x}(\Delta z), \ldots, a_{\Delta x}(\mathrm{L}\Delta z), a_{2\Delta x}(\Delta z), \ldots, a_{2\Delta x}(\mathrm{L}\Delta z), \ldots, a_{\mathrm{W}\Delta x}(\Delta z), \ldots, a_{\mathrm{W}\Delta x}(\mathrm{L}\Delta z)\right)^T$$

[Math 164]

$$\mathcal{A}_{Re} = \left(\mathcal{A}_{Re\,j,1,1}, \ldots, \mathcal{A}_{Re\,j,1,L}, \mathcal{A}_{Re\,j,2,1}, \ldots, \mathcal{A}_{Re\,j,2,L}, \mathcal{A}_{Re\,j,W,1}, \ldots, \mathcal{A}_{Re\,j,W,L}\right)$$

[Math 165]

$$\mathcal{A}_{Im} = \left(\mathcal{A}_{Im\,j,1,1}, \ldots, \mathcal{A}_{Im\,j,1,L}, \mathcal{A}_{Im\,j,2,1}, \ldots, \mathcal{A}_{Im\,j,2,L}, \mathcal{A}_{Im\,j,W,1}, \ldots, \mathcal{A}_{Im\,j,W,L}\right)$$

[Math 166]

$$\mathcal{A}_{Re\,j,q,k} = 4Re(A_R)Re\left(a_{Ref\,q\Delta x}(k\Delta z)\right) \int_{(k-1)\Delta z}^{k\Delta z} \left(S13_{jq}(z) + iS24_{jq}(z)\right) dz$$

[Math 167]

$$\mathcal{A}_{Re\,j,q,k} = 4Im(A_R)Im\left(a_{Ref\,q\Delta x}(k\Delta z)\right) \int_{(k-1)\Delta z}^{k\Delta z} \left(S13_{jq}(z) + iS24_{jq}(z)\right) dz$$

Et où $A_{Re}$ et $A_{Im}$ sont deux matrices de M lignes et L*W colonnes chacune telles que :

[Math 164]

$$\mathcal{A}_{Re} = \left(\mathcal{A}_{Re\,j,1,1}, \ldots, \mathcal{A}_{Re\,j,1,L}, \mathcal{A}_{Re\,j,2,1}, \ldots, \mathcal{A}_{Re\,j,2,L}, \mathcal{A}_{Re\,j,W,1}, \ldots, \mathcal{A}_{Re\,j,W,L}\right)$$

La figure 9 illustre schématiquement la représentation matricielle de l'opération de reconstruction du profil d'absorption bidimensionnel $a_{xz}$.
La fonction de coût associée à la reconstruction du vecteur $a_{xz}$ peut être de la même forme que dans l'exemple de la figure 5 (équation Math 42), avec un terme de fidélité de même nature et un terme de régularisation permettant de contraindre la reconstruction 2D de l'absorption du milieu.
Ce principe de mesure en deux dimensions a été présenté pour un mode de détection synchrone avec démodulation dans le domaine électronique. Il pourra toutefois aisément être adapté à un mode d'acquisition basse fréquence avec démodulation dans le domaine optique, tel que décrit en relation avec la figure 6.
De plus, ce principe peut être étendu à des mesures en trois dimensions en ajoutant un axe y, par exemple orthogonal aux axes x et z, et en utilisant une matrice de transducteurs ultrasonores à deux dimensions et une fonction d'activation γ dépendant de x et de y.
Afin d'augmenter la résolution latérale (en x et/ou en y) de la mesure, on pourra de plus utiliser une barrette ou matrice de transducteurs et prévoir une activation simultanée de groupes glissants recouvrants de plusieurs transducteurs voisins.
On pourra de plus prévoir d'utiliser des fonctions de modulation Φ différentes en fonction des transducteurs activés, et non plus une fonction de modulation commune à l'ensemble des transducteurs.
Plus généralement, on pourra activer séquentiellement des sous-groupes de transducteurs en fonction des formes d'excitation acoustiques souhaitées (selon des directions variables par exemple dans un plan ou dans un volume donné), afin d'accroître la résolution latérale.

On a proposé ci-dessus un modèle d'acquisition permettant de reconstruire l'amplitude du profil d'absorption $a_M$ du faisceau objet. Ce vecteur, fonction de la position z ($a_M(z)$), a jusqu'à présent été considéré comme ayant une phase constante en fonction de l'indice et de la position de mesure (c'est-à-dire comme si l'on considérait un unique pixel mesurant un milieu statique).

En notant désormais :

[Math 168]

$$a_M(z) = \rho(z)e^{i\theta^*(z)}$$

on cherche à reconstruire le profil d'absorption $\rho$ qui est un vecteur constitué de valeurs réelles positives comprises entre 0 et 1.

En considérant désormais une matrice de $n_v$ par $n_h$ pixels échantillonnant des grains de speckle (c'est-à-dire des taches de la figure d'interférence), on mesure simultanément $n_v*n_h$ différentes variantes du coefficient $Y_j$. En supposant que le signal reçu par chaque pixel a sa propre phase aléatoire $\theta$ uniformément répartie entre 0 et $2\pi$, on se ramène à un profil $a_M$ dépendant de l'indice p du pixel, soit :

[Math 169]

$$a_M(p,z) = \rho(z)e^{i\theta^*(p,z)}$$

On peut donc considérer la mesure du coefficient $Y_j$ comme un ensemble de $n_v*n_h$ échantillons modélisés par le produit scalaire des matrices de passage définies précédemment avec le profil $a_M$ tel que :

[Math 170]

$$Y_j[p] = \langle (\mathcal{A}_{Re})_j, Re(a_M[p]) \rangle + \langle (\mathcal{A}_{Im})_j, Im(a_M[p]) \rangle$$

[Math 171]

$$Y_j[p] = \langle (\mathcal{A}_{Re})_j, \rho \odot \cos(\theta[p]) \rangle + \langle (\mathcal{A}_{Im})_j, \rho \odot \sin(\theta[p]) \rangle$$

où le symbole [Math 172] $\odot$ désigne la multiplication point à point.

On a donc :

[Math 173]

$$Y_j[p] = \langle (\mathcal{A}_{Re})_j \odot \cos(\theta[p]), \rho \rangle + \langle (\mathcal{A}_{Im})_j \odot \sin(\theta[p]), \rho \rangle$$

[Math 174]

$$Y_j[p] = \langle (\mathcal{A}_{Re})_j \odot \cos(\theta[p]) + (\mathcal{A}_{Im})_j \odot \sin(\theta[p]), \rho \rangle$$

[Math 175]

$$Y_j[p] = \langle\, Re\big((\mathcal{A}_{Re})_j \odot \cos(\theta[p]) + (\mathcal{A}_{Im})_j \odot \sin(\theta[p])\big), \rho\,\rangle$$
$$+ i\langle\, Im\big((\mathcal{A}_{Re})_j \odot \cos(\theta[p]) + (\mathcal{A}_{Im})_j \odot \sin(\theta[p])\big), \rho\,\rangle$$

[Math 176]

$$Y_j[p] = \langle\, \beta_{Re}[p], \rho\,\rangle + i\langle\, \beta_{Im}[p], \rho\,\rangle$$

avec :

[Math 177]

$$\beta_{Re\,j}[p] = Re\big((\mathcal{A}_{Re})_j\big) \odot \cos(\theta[p]) + Re\big((\mathcal{A}_{Im})_j\big) \odot \sin(\theta[p])$$

[Math 178]

$$\beta_{Im\,j}[p] = Im\big((\mathcal{A}_{Re})_j\big) \odot \cos(\theta[p]) + Im\big((\mathcal{A}_{Im})_j\big) \odot \sin(\theta[p])$$

En considérant que la phase θ suit une loi uniforme entre 0 et 2π, la variance suivant l'axe des pixels, notée Var$_p$, s'exprime comme suit :

[Math 179]

$$Var_p\left(\beta_{Re\,j}\right) = \frac{Re\big((\mathcal{A}_{Re})_j\big)^2 + Re\big((\mathcal{A}_{Im})_j\big)^2}{2}$$

[Math 180]

$$Var_p\left(\beta_{Im\,j}\right) = \frac{Im\big((\mathcal{A}_{Re})_j\big)^2 + Im\big((\mathcal{A}_{Im})_j\big)^2}{2}$$

On note alors :

[Math 181]

$$\sigma_p\left(\beta_{Re\,j}\right) = \sqrt{\frac{Re\big((\mathcal{A}_{Re})_j\big)^2 + Re\big((\mathcal{A}_{Im})_j\big)^2}{2}}$$

[Math 182]

$$\sigma_p\left(\beta_{Im_j}\right) = \sqrt{\frac{Im\left((\mathcal{A}_{Re})_j\right)^2 + Im\left((\mathcal{A}_{Im})_j\right)^2}{2}}$$

[Math 183]

$$\sigma_p(Y_j) = \langle\sigma_p\left(\beta_{Re_j}\right), \rho\rangle + i\langle\sigma_p\left(\beta_{Im_j}\right), \rho\rangle$$

[Math 184]

$$\widehat{Y_j} = \langle\widehat{\beta_{Re_j}}, \rho\rangle + i\langle\widehat{\beta_{Im_j}}, \rho\rangle$$

[Math 185]

$$\widehat{Y} = \widehat{B_{Re}}\,\rho + i\,\widehat{B_{Im}}\,\rho = \left(\widehat{B_{Re}} + i\,\widehat{B_{Im}}\right)\rho$$

On dispose donc d'un modèle de mesure spécifiant que la variance des signaux mesurés par les p pixels pour chaque acquisition j est une fonction des matrices de passage identifiées précédemment. Ce modèle peut être exploité pour reconstruire le module ρ du profil d'absorption.

Divers modes de réalisation et variantes ont été décrits. L'homme de l'art comprendra que certaines caractéristiques de ces divers modes de réalisation et variantes pourraient être combinées, et d'autres variantes apparaîtront à l'homme de l'art. En particulier, les modes de réalisation décrits ne se limitent pas aux formules mathématiques employées dans la présente description. Plus généralement, la méthode proposée est applicable pour d'autres formes d'équations modélisant le processus optique. A titre d'exemple, dans le modèle d'acquisition présenté ci-dessus, on a considéré une amplitude $a_M$ commune à l'ordre ωL+ωUS et à l'ordre ωL-ωUS. A titre de variante, le modèle peut être affiné en considérant un vecteur propre pour chacun des deux ordres.

De plus, dans les exemples décrits ci-dessus, il est prévu un unique saut de phase par période de modulation acoustique $T_{US}$. A titre de variante, on peut prévoir un nombre de sauts de phase par période $T_{US}$ supérieur à 1, pour améliorer la sélectivité spatiale lors de la reconstruction du profil d'absorption.

Par ailleurs, les exemples décrits ci-dessus peuvent être adaptés en remplaçant les sauts de phase appliqués par les dispositifs de modulation et de démodulation par des sauts de fréquence ou des sauts d'amplitude.

En outre, dans les exemples décrits ci-dessus, le terme $a_{Mref}$ pourra être utilisé pour prendre en compte, dans la matrice de passage, une éventuelle non uniformité du faisceau acoustique de marquage de l'échantillon dans la zone de marquage.

Les méthodes de mesure de profil d'absorption décrites ci-dessus peuvent de plus être combinées avec une méthode de conjugaison de phase afin d'obtenir un meilleur rapport signal à bruit. Chaque mesure se fait alors en trois étapes :

1) Mesure du vecteur $Y_j$ par toute méthode adaptée, éventuellement une méthode connue de l'état de l'art ;
2) Extraction de la phase de la mesure complexe $Y_j$ puis refocalisation du faisceau incident sur l'échantillon par conjugaison de phase, par exemple au moyen d'un dispositif du type décrit dans la demande FR1903526 (B18214/DD19189) précédemment déposée par le demandeur ; et
3) Nouvelle mesure du vecteur $Y_{j+1}$ selon la méthode décrite ci-dessus.

Dans une autre variante, une série de mesures peut être réalisée avec un même couple de motifs Ω et Φ pour chaque mesure, une analyse de la variation de la phase de ces mesures étant mise en oeuvre pour extraire des informations quant au milieu observé. En particulier, en champ libre, les variations de phase peuvent permettre de détecter des mouvements dans l'axe du faisceau, ou encore des variations de densité d'un milieu diffusant (par exemple le brouillard). En milieu diffusant, les variations de phase peuvent permettre de détecter d'éventuels mouvements des diffuseurs dans le milieu (variation des mouvements de plaquettes permettant de remonter à des informations de rythme cardiaque par exemple). La fonction de coût sera éventuellement adaptée en conséquence (le terme de régularisation pondéré par le coefficient λ peut s'appliquer sur le module ou la phase des mesures).

Par ailleurs, on pourra prévoir un apprentissage de motifs d'absorption avec les motifs Ω et Φ pour permettre une détection directe des profils d'absorption à partir des mesures, sans passer par la phase de reconstruction par minimisation de la fonction de coût.

On notera de plus que bien que, dans les exemples décrits ci-dessus, la modulation du faisceau lumineux objet et, le cas échéant, la modulation du faisceau lumineux de référence, soient réalisées par modulation acousto-optique, les modes de réalisation décrits ne se limitent pas à ce cas particulier. Plus généralement, toute autre méthode de modulation d'un faisceau lumineux peut être utilisée, par exemple des méthodes de modulation par effet Pockels. En particulier, dans les exemples des figures 6 et 7, les modulateurs acousto-optiques MAO1 et MAO2 peuvent être remplacés par des modulateurs à effet Pockels. Dans ce cas, le signal de modulation est un champ électrique et non plus une onde acoustique.

**Revendications**

1. Système d'imagerie, comportant :

   - une source de lumière cohérente (101) fournissant un faisceau objet (O) et un faisceau de référence (R) ;
   - un dispositif de modulation (120 ; MAO1 ; 820) adapté à moduler tout ou partie du faisceau objet par un signal de modulation ;
   - un capteur d'images (109 ; 609) agencé pour recevoir une figure d'interférence résultant d'une combinaison du faisceau objet (O) et du faisceau de référence (R) ; et
   - un dispositif de démodulation (109 ; MAO2) de la partie modulée du faisceau objet,

   le système étant configuré pour, lors d'une phase de mesure :

   - appliquer au signal de modulation, par l'intermédiaire du dispositif de modulation (120 ; MAO1 ; 820), une première séquence pseudo-aléatoire (Φ) de sauts d'un paramètre choisi parmi la phase, la fréquence et l'amplitude ; et
   - appliquer simultanément à la partie modulée du faisceau objet (O), par l'intermédiaire du dispositif de démodulation (109 ; MAO2), une deuxième séquence pseudo-aléatoire (Ω) de sauts dudit paramètre,

   dans lequel les première (Φ) et deuxième (Ω) séquences de sauts dudit paramètre sont non corrélées.

2. Système selon la revendication 1, configuré pour, lors d'une phase d'acquisition, mettre en oeuvre M phases de mesure successives, M étant un entier supérieur ou égal à 2, et, à chaque phase de mesure, acquérir, via le capteur d'images (109 ; 609), une valeur ($Y_j$) représentative du champ complexe de la partie modulée du faisceau objet (O).

3. Système selon la revendication 2, comportant un dispositif de traitement configuré pour mettre en oeuvre une étape de reconstruction, à partir des M valeurs ($Y_j$) acquises lors de la phase d'acquisition, d'un jeu de L valeurs représentatives de l'absorption lumineuse à L positions distinctes sur le trajet optique de la partie modulée du faisceau objet, L étant un entier supérieur ou égal à 2.

4. Système selon la revendication 3, dans lequel L est supérieur à M.

5. Système selon l'une quelconque des revendications 1 à 4, dans lequel le dispositif de modulation (120 ; MAO1 ; 820) est un dispositif de modulation acousto-optique.

6. Système selon la revendication 5, dans lequel le dispositif de modulation (120 ; 820) comprend un transducteur ultrasonore, le signal de modulation étant une onde ultrasonore appliquée par le transducteur ultrasonore à une partie d'un échantillon (105) à analyser, placé sur le trajet optique du faisceau objet (0).

7. Système selon la revendication 6, dans lequel le dispositif de modulation (820) comprend une pluralité de transducteurs ultrasonores agencés en barrette ou en matrice, et dans lequel, lors de chaque phase de mesure, l'onde ultrasonore de modulation est émise par fraction par les différents transducteurs selon une fonction pseudo-aléatoire γ.

8. Système selon la revendication 4, dans lequel le dispositif de modulation (MAO1) comprend un modulateur acousto-optique placé sur le trajet optique du faisceau objet (O) en amont d'une scène (705) à analyser.

9. Système selon l'une quelconque des revendications 1 à 8, dans lequel le dispositif de démodulation est un dispositif de démodulation électronique intégré au capteur d'images (109) .

10. Système selon l'une quelconque des revendications 1 à 8, dans lequel le dispositif de démodulation comprend un modulateur acousto-optique (MAO2) placé sur le trajet optique du faisceau de référence (R), en amont du capteur d'images (609).

**Patentansprüche**

1. Ein Abbildungssystem, das Folgendes aufweist:

- eine kohärente Lichtquelle (101), die einen Objektstrahl (O) und einen Referenzstrahl (R) liefert;
- eine Modulationsvorrichtung (120; MAO1; 820), die in der Lage ist, den gesamten oder einen Teil des Objektstrahls mit einem Modulationssignal zu modulieren;
- einen Bildsensor (109; 609), der angeordnet ist, um ein Interferenzmuster zu empfangen, das aus einer Kombination des Objektstrahls (O) und des Referenzstrahls (R) resultiert; und
- eine Vorrichtung (109; MAO2) zur Demodulation des modulierten Teils des Objektstrahls,

wobei das System konfiguriert ist während einer Messphase zum:
Anlegen an das Modulationssignal, über die Modulationsvorrichtung (120; MAO1; 820), einer ersten Pseudozufalls-folge ($\Phi$) von Sprüngen eines Parameters, der aus der Phase, der Frequenz und der Amplitude ausgewählt ist; und

- gleichzeitiges Anlegen an den modulierten Teil des Objektstrahls (O), über die Demodulationsvorrichtung (109; MAO2), eine zweite Pseudozufallsfolge ($\Omega$) von Sprüngen des Parameters,

wobei die erste ($\Phi$) und die zweite ($\Omega$) Folge von Sprüngen des Parameters nicht korreliert sind.

2. System nach Anspruch 1, das konfiguriert während einer Erfassungsphase zum Implementieren von M aufeinanderfolgenden Messphasen, wobei M eine ganze Zahl größer oder gleich 2 ist, und in jeder Messphase zum Erfassen, über den Bildsensor (109; 609), eines Werts ($Y_j$), der für das komplexe Feld des modulierten Teils des Objektstrahls (O) repräsentativ ist.

3. System nach Anspruch 2, aufweisend eine Verarbeitungsvorrichtung, die konfiguriert ist zum Implementieren eines Rekonstruktionsschritts, basierend auf den während der Erfassungsphase erfassten M Werten ($Y_j$), eines Satzes von L Werten, die für die Lichtabsorption an L verschiedenen Positionen im optischen Pfad des modulierten Abschnitts des Objektstrahls repräsentativ sind, wobei L eine ganze Zahl größer als oder gleich 2 ist.

4. System nach Anspruch 3, wobei L größer als M ist.

5. System nach einem der Ansprüche 1 bis 4, wobei die Modulationsvorrichtung (120; MAO1; 820) eine akustisch-optische Modulationsvorrichtung ist.

6. System nach Anspruch 5, wobei die Modulationsvorrichtung (120; 820) einen Ultraschallwandler aufweist, wobei das Modulationssignal eine Ultraschallwelle ist, die von dem Ultraschallwandler an einen Teil einer zu analysierenden Probe (105) angelegt wird, die im optischen Pfad des Objektstrahls (O) angeordnet ist.

7. System nach Anspruch 6, wobei die Modulationsvorrichtung (820) eine Vielzahl von Ultraschallwandlern aufweist, die in einem Array, linear oder nicht, angeordnet sind, und während jeder Messphase die Ultraschallmodulationswelle in Anteilen von den verschiedenen Wandlern gemäß einer Pseudozufallsfunktion $\gamma$ emittiert wird.

8. System nach Anspruch 4, wobei die Modulationsvorrichtung (MAO1) einen akustisch-optischen Modulator aufweist, der im optischen Pfad des Objektstrahls (O) stromaufwärts bezüglich einer zu analysierenden Szene (705) angeordnet ist.

**9.** System nach einem der Ansprüche 1 bis 8, wobei die Demodulationsvorrichtung eine in den Bildsensor (109) integrierte elektronische Demodulationsvorrichtung ist.

**10.** System nach einem der Ansprüche 1 bis 8, wobei die Demodulationsvorrichtung einen akustisch-optischen Modulator (MAO2) aufweist, der im optischen Pfad des Referenzstrahls (R) stromaufwärts bezüglich des Bildsensors (609) angeordnet ist.

**Claims**

**1.** An imaging system, comprising:

- a coherent light source (101) delivering an object beam (O) and a reference beam (R);
- a modulation device (120; MA01; 820) capable of modulating all or part of the object beam with a modulation signal;
- an image sensor (109; 609) arranged to receive an interference pattern resulting from a combination of the object beam (O) and of the reference beam (R) ; and
- a device (109; MAO2) of demodulation of the modulated portion of the object beam,

the system being configured to, during a measurement phase:

- apply to the modulation signal, via the modulation device (120; MA01; 820), a first pseudo-random sequence ($\Phi$) of jumps of a parameter selected among the phase, the frequency, and the amplitude; and
- simultaneously apply to the modulated portion of the object beam (O), via the demodulation device (109; MAO2), a second pseudo-random sequence ($\Omega$) of jumps of said parameter,

wherein the first ($\Phi$) and second ($\Omega$) sequences of jumps of said parameter are non-correlated.

**2.** The system according to claim 1, configured to, during an acquisition phase, implement M successive measurement phases, M being an integer greater than or equal to 2, and, at each measurement phase, acquire, via the image sensor (109; 609), a value ($Y_j$) representative of the complex field of the modulated portion of the object beam (O) .

**3.** The system according to claim 2, comprising a processing device configured to implement a step of reconstruction, based on the M values ($Y_j$) acquired during the acquisition phase, of a set of L values representative of the light absorption at L different positions on the optical path of the modulated portion of the object beam, L being an integer greater than or equal to 2.

**4.** The system according to claim 3, wherein L is greater than M.

**5.** The system according to any of claims 1 to 4, wherein the modulation device (120; MA01; 820) is an acoustic-optical modulation device.

**6.** The system according to claim 5, wherein the modulation device (120; 820) comprises an ultrasound transducer, the modulation signal being an ultrasound wave applied by the ultrasound transducer to a portion of a sample (105) to be analyzed, placed on the optical path of the object beam (O).

**7.** The system according to claim 6, wherein the modulation device (820) comprises a plurality of ultrasound transducers arranged in an array, linear or not, and, during each measurement phase, the ultrasound modulation wave is emitted in fractions by the different transducers according to a pseudo-random function $\gamma$.

**8.** The system according to claim 4, wherein the modulation device (MA01) comprises an acoustic-optical modulator placed on the optical path of the object beam (O) upstream of a scene (705) to be analyzed.

**9.** The system according to any of claims 1 to 8, wherein the demodulation device is an electronic demodulation device integrated to the image sensor (109).

**10.** The system according to any of claims 1 to 8, wherein the demodulation device comprises an acoustic-optical modulator (MAO2) placed on the optical path of the reference beam (R), upstream of the image sensor (609).

**Fig 1**

**Fig 2**

Fig 3

Fig 4

Fig 5

**Fig 6**

**Fig 7**

Fig 8

Fig 9

## RÉFÉRENCES CITÉES DANS LA DESCRIPTION

**Documents brevets cités dans la description**

- US 2016327904 A

- FR 1903526